# EUROPEAN PATENT SPECIFICATION

(11) **EP 4 445 121 B1**
(45) Date of publication and mention of the grant of the patent: **14.10.2026**
(21) Application number: 22822203.0
(22) Date of filing: 07.12.2022
(51) Int. Cl.: G01N 21/65, G01N 21/21, G01N 21/64

(54) **MULTI-EXCITATION RAMAN SPECTROSCOPY METHOD AND APPARATUS**
VERFAHREN UND VORRICHTUNG FÜR RAMAN-SPEKTROSKOPIE MIT MEHRFACHERREGUNG
PROCÉDÉ ET APPAREIL DE SPECTROSCOPIE RAMAN À EXCITATION MULTIPLE

(30) Priority: 08.12.2021 GB 202117705
(43) Date of publication of application: 16.10.2024
(73) Proprietor: University of Southampton, Southampton, Hampshire SO17 1BJ (GB)
(72) Inventor: MAHAJAN, Sumeet, Southampton Hampshire SO17 1BJ (GB); WEBB, Jeremy, Southampton Hampshire SO17 1BJ (GB); LISTER, Adam, Southampton Hampshire SO17 1BJ (GB); HIGHMORE, Callum, Southampton Hampshire SO17 1BJ (GB); DEVITT, George, Southampton Hampshire SO17 1BJ (GB); HANRAHAN, Niall, Southampton Hampshire SO17 1BJ (GB)
(74) Representative: J A Kemp LLP
(86) International application number: PCT/GB2022/053117
(87) International publication number: WO 2023/105219

(56) References cited:
- CN-A- 111 256 821
- US-A1- 2011 087 439
- GRUN JACOB ET AL: "Identification of Bacteria from Two-Dimensional Resonant-Raman Spectra", ANALYTICAL CHEMISTRY, vol. 79, no. 14, 9 June 2007 (2007-06-09), US, pages 5489 - 5493, XP055946383, ISSN: 0003-2700, DOI: 10.1021/ac070681h
- SMITH ZACHARY J. ET AL: "Surface-sensitive polarized Raman spectroscopy of biological tissue", OPTICS LETTERS, vol. 30, no. 11, 1 June 2005 (2005-06-01), US, pages 1363 - 1365, XP055946656, ISSN: 0146-9592, DOI: 10.1364/OL.30.001363

## Description

The invention relates to methods and apparatuses for performing Raman spectroscopy, in particular for detection of contaminants in a sample.

Current methods of diagnosis of bacterial infections are complex, skill-intensive, and can take days from patient sample to result. This places a significant burden on healthcare providers, delays treatment and can lead to adverse patient outcomes. Most methods that are used or being developed utilise traditional culture techniques to increase the number of bacteria available, and thus require an overnight incubation of the sample at the minimum. This is primarily due to diagnostic samples comprising of complex biological materials and heterogeneous microbial communities. This can then be followed by Fluorescence In-Situ Hybridisation (FISH) microscopy for biofilms , mass spectrometry, or Polymerase Chain Reaction (PCR). These techniques require dedicated laboratory facilities, specialist personnel, and take a long time (typically 48 hours) to return a diagnosis. The methodologies used are therefore labour, cost, and time intensive, delaying effective treatment by days.

For complex clinical biofilm infections, it can take days from collecting and processing a patient's sample to achieving a result. This can be a significant issue in the event of time-sensitive medical situations such as sepsis, where a causative pathogen and information about drug-resistant genotypes can be invaluable.

Improved and cost-effective screening of bacterial genotypes would also facilitate monitoring the emergence of drug resistance in other clinical settings, and help with drug stewardship. This process is vital to combatting the rise of drug resistant pathogens, such as C. *difficile* and S. *aureus,* which kill large numbers of people, and present a large financial burden on healthcare providers.

In existing mainstream techniques, samples from patients are cultured in a broth media, and then a portion of this is sub-cultured again on agar media. Colonies can then be picked for identification by scientific staff and any desired tests for antimicrobial resistance (AMR) can be conducted. These tests suffer from low sensitivity, and growth can be inhibited by contaminants, and are slow, with results taking between 24 and 72 hours. Agar media used are routinely specialised, selective media, which are expensive and useful only for isolating known bacterial species or subspecies of interest, to the exclusion of any other bacterium in a sample. Additionally, viable but non-culturable bacteria (VBNCs) are unable to be detected by these methods because they simply do not grow in standard media. The lengthy time to return results has been shortened by the use of peptide nucleic acid probes in Fluorescence *in-situ* Hybridisation microscopy (PNA-FISH), which has been used to directly identify *Staphylococcus, Enterococcus, Klebsiella,* and *Candida* bacteria directly from positive blood culture in under two hours. However, this still requires a both culture step, which lengthens this analysis, and requires the use of probes specific to each target species.

Molecular techniques have also been developed for the purpose of pathogen identification. In particular, polymerase chain reaction (PCR) and nucleic acid sequencing technologies have received wide interest. These techniques are faster than conventional culture techniques, and have improved sensitivity and specificity. However, matrix contaminants can inhibit amplification of DNA, and analyses cannot differentiate viable and non-viable cells, as both contain DNA; this issue is sometimes exacerbated by the use of a culture-based pre-enrichment step to amplify bacterial signal at the cost of the veracity of the sample. Further, samples require extensive preparation and manipulation. Further, systems designed for use in blood samples offer poor sensitivity and selectivity, as well as providing no information about AMR.

A derivative of conventional DNA-DNA hybridisation has also been developed to help counteract the laborious nature of the conventional whole genome DNA-DNA method, and the impossibility of centralising a database. This method uses random DNA fragments in a microarray to identify bacteria. The multitude of wells (>1000) allows broad identification capacity, but required the use of exogenous fluorescent labels to image the binding which adds sample preparation complexity and lengthens analyses. A nonelectrophoretic bioluminometric DNA sequencing method called pyrosequencing has been developed and used for some years, and is based on detection of the nucleotides being incorporated onto a DNA chain by DNA polymerase. This is done by light detection of a chain reaction when pyrophosphate is released. This technique has been developed to work in microfluidic systems to reduce material costs and improve read length, but has not yet been multiplexed to increase throughput.

The use of using DNA sequencing on the 16S rRNA for bacterial identification provides a robust technique with the considerable advantage of not having to target any specific known bacterium beforehand. However, high similarities in 16S sequence between some different bacterial species or strains, for example in newly diverged species, can limit the utility of 16S sequencing. Increasingly, micro-organisms can be identified using metagenomics, in which the DNA of each organism in a polymicrobial sample can be sequenced. The 16S method can be applied to metagenomics studies, for example in identifying pathogens present in bacterial bloodstream infections. The metagenomics use of 16S sequencing facilitates detection of a broad range of species, but the specificity at lower taxonomic levels is limited. More thorough metagenomics approaches require the extraction of genomic DNA of an entire polymicrobial sample. This permits the identification of any bacteria within a sample, without the limitations that come with only using the 16S sequences, and can be carried out on complex environmental or clinical samples. While this methodology can provide exhaustive microbial identification and rapid next generation sequencing technologies are continually being developed, it is expensive and extremely labour intensive and produces vast datasets which can exceed necessity. Furthermore, biochemical studies are unable to attribute clinical significance to any particular bacterial species and exclude any phenotypic data about the sample. For this reason, the use of diagnostic metagenomics can be complemented by traditional culture techniques.

Electrochemical methods have also received much focus as alternative platform for the detection of pathogens, as many of them are label-free, and offer greater sensitivity and lower cost than current methods. Their label free nature offers potential for direct detection and provides ease of use. These devices also offer the potential of being wearable or implantable, as they may be flexible and miniaturisable. However, whilst glucose biosensors are now on the market, electrochemical sensors for real time monitoring of pathogens are not. In large part, this arises from the current limitations in terms of sample preparation steps, times for analysis, and sensitivity. These sensors can take several approaches to detection of pathogens. One implementation achieved semi-direct detection of two pathogenic species based on the presence of the microbial enzyme, cytochrome c oxidase. Because of the ubiquity of this enzyme, this rapid detection lacked specificity. Redox mediated detection of *E. coli* was also demonstrated using TMPD as a redox mediator interacting with cytochrome c oxidase using impact electrochemistry to achieve detection within minutes. As alternatives to microbial enzymes, it is possible to achieve detection via sensing of DNA (via the binding of pathogen gene fragments) or cellular metabolites, such as pyocyanin and pyoverdine.

Mass spectrometry (MS) has also been applied to the identification of bacteria down to the strain level, often by elucidation of the bacterial proteome. MS has been widely studied for this application, and excellent reviews exist discuss its ability as a powerful tool for the strain level characterisation of bacteria, such as the one by Sandrin *et al,* which deals with the application of Matrix Assisted Laser Desorption Ionisation (MALDI TOF) MS for this application. MS can return results within an hour after an initial broth culture of the sample has been grown, and offers a qualitative result based on the detection of a broad range of molecules present within the sample at different concentrations. MALDI TOF MS, Electrospray Ionisation MS, and Desorption Electrospray Ionisation MS, have all been described as methods for the identification and analysis of clinical pathogens. Despite its power, MS is not without drawbacks. The equipment is expensive and requires dedicated laboratory space as it does not miniaturise well. It is also complex, and requires trained operators to use effectively. Matching of mass spectra also requires the building of a library, which is not without issues. Studies on E. *coli* by MALDI MS have highlighted discrepancies in the ions identified in mass spectra, likely arising from differences in methodology, such as machine parameters, sample preparation and different sample matrices, were mentioned by Wunschel *et al* in their interlaboratory comparison of bacterial analysis using MALDI TOF MS. Additionally, the dynamic nature of the bacterial proteome can hinder microbial identification as it is highly dependent on its environment. Whilst MS methods undeniably produce incredibly information-rich data that can be compared against libraries produced predicted masses from proteomic databases, this lack of standardisation could present issues for MS-based diagnostic libraries of common pathogens of interest.

Therefore, while a range of possible diagnostic techniques exist and are under investigation, most still possess similar drawbacks of high labour costs, or the use of expensive materials and complex apparatus.

The shortcomings in medical diagnostic capabilities are exemplified in cystic fibrosis (CF), where diagnosis of infection relies on the manipulation of complex sputum samples. CF is an autosomal recessive genetic disorder, affecting 1.37 births per 10,000 in the UK. Affected individuals are extremely susceptible to bacterial infection of the lower respiratory tract due to impaired innate immune function in the lung and the consequent overproduction of mucus. Even with the advent of new disease modifying treatment, recurrent infections cause a dramatic reduction of quality and length of life [44]. The pathophysiology of the CF lung impedes the administration of effective treatment. Intensive antibiotic treatment is reported to be beneficial in eradicating Pseudomonas aeruginosa at its early stages of colonisation. When established in the CF lung, there is evidence that P. aeruginosa exists as biofilms, microbial communities protected by a self-produced polymeric matrix. The protection afforded to bacteria by a biofilm reduces the efficacy of antibiotic treatment, and is a driver of antimicrobial resistance, so effective and rapid treatment is of great importance. In CF, P. aeruginosa lineages display variation in virulence and antibiotic sensitivity and undergo recombination events to further increase phenotypic diversity and antibiotic resistance. Taken together, these attributes necessitate a rapid and specific alternative to the current slow and resource intensive methods of diagnosing infection in CF patients.

Another area where improvement in diagnostic techniques is needed is neurodegenerative diseases. For neurodegenerative diseases and dementias, there are currently no methods available for early or presymptomatic detection. Current methods based on MRI/PET or CT are suited only for late stage detection. There are a few biomarkers identified in cerebrospinal fluid whose analysis is done by combination of biochemical and molecular techniques. However, the single or few biomarker approach has been found to be inadequate for diagnosis and currently does not lend itself to early detection of the disease. Biofluid-based methods of detection, especially that rely on holistic biomarkers, are completely lacking in the current diagnostics landscape.

Raman spectroscopy is a label-free vibrational spectroscopy technique that relies on the inelastic scattering of light to probe the molecular vibrations present in a sample. This allows development of a specific molecular fingerprint that can be used to identify molecular, biotic and abiotic components in a sample. Raman spectroscopy offers many advantages in terms of time, cost, and complexity of analysis over resource intensive culture based methods and biological assays, as well as other techniques such as enzyme-linked immunosorbent assays (ELISA), mass spectrometry, fluorescence, and infrared spectroscopy. Acquisition of spectra is rapid, reagentless, and requires no labelling or other complex sample-preparation steps. It is also water-insensitive, which is an advantage in biological analyses. Raman spectroscopy of bacterial samples requires no culturing step, offering a considerable time saving over conventional analyses. Results are available within minutes, while culture methods typically require 24-48 hours before detection of a specific biomarker can be achieved.

Raman spectroscopy has been applied to the microbiology of complex biological and clinical samples, e.g. the detection of human pathogens inoculated into ascetic fluid [1]. Kloß *et al* were able to characterise respiratory pathogens at the species level using Raman spectroscopy [2], and Ghebremedhin *et al* used the technique to differentiate between 31 clinical isolates of *Acinetobacter baumannii* at the strain level [3]. However, this can often involve long acquisition times that slow down analysis because of the relative scarcity of Raman-scattering events.

Surface-Enhanced Resonant Raman scattering (SERS) offers advantages over spontaneous Raman in terms of speed and an improved limit of detection due to enhancement of signals by electromagnetic and/or chemical mechanisms typically observed with nanoscale metallic materials. SERS has been used to distinguish *Escherichia coli* isolates based on their sensitivity to carbapenem antibiotics [4], and to identify common CF pathogens in pellets with silver nanoparticles [5], and to map *P. aeruginosa* colonies by acquiring SER spectra via laser-scanning and using the intensity of a C-H bond associated with pyocyanin [6-10]. Despite the capabilities and advantages of SERS, it requires the introduction of exogenous materials, such as metallic nanoparticles, to the sample to provide signal enhancement. Additionally, this exogenous material must interact closely and reproducibly with the analyte, and the reproducibility and sensitivity can be severely affected by any variations in the number or intensity of plasmonic hotspots, or from changes to the frequency of the plasmon resonance arising from different degrees of aggregation, as might be seen with colloidal gold nanoparticles.

Therefore, while Raman spectroscopy has numerous advantages over traditional techniques, it is clear that is still has drawbacks that can prevent it being as effective as required in clinical diagnostic applications.

Improved detection and diagnosis technology could be applied beyond microbial detection to diagnosis of any disease or condition wherein distinction and classification of species is needed. Such improved technology would also have applications other than in the healthcare sector as well. In the food production industry, identification of contaminating bacteria within products is of great concern, and a rapid techniques for testing samples for bacterial contamination would be of great value. Other possible applications include the oil industry, where biofouling inside pipelines is a concern.

According to a first aspect of the invention, there is provided a method of identifying one or more substances in a sample according to claim 1.

By using multiple different excitation modes to measure the sample, combining the data from all of the excitation modes, and using contributions from plural photophysical processes, the information available for distinguishing between species to be analysed is greatly increased. The method therefore provides improved ability to distinguish different species and sub-species, while also allowing for faster sample analysis from using an optical technique.

In some embodiments, identifying the one or more substances comprises using a multivariate analysis. For example, the multivariate analysis may comprise principal component analysis or linear discriminant analysis. Using a multivariate analysis allows for the effects of multiple different measurement variables to be accounted for in a single, cohesive analysis. Principal component analysis and linear discriminant analysis are two well-understood techniques that have been found to be effective in this application.

In some embodiments, identifying the one or more substances comprises combining the measured spectra using the multivariate analysis to obtain a multi-dimensional signature of the sample. A multi-dimensional signature can be measured for the sample, which allows for a standardised mechanism for the comparison of different samples.

In some embodiments, identifying the one or more substances further comprises comparing the multi-dimensional signature to one or more reference signatures. Comparing the measured signature to a reference signature, which may have been measured for a reference sample of known species and sub-species, allows a particular species (or sub-species) to be identified in the sample under consideration.

In some embodiments, identifying the one or more substances comprises using a machine-learning algorithm. For example, the machine-learning algorithm may comprise a support vector machine or a neural network. Using a machine-learning algorithm can be an efficient way to determine how samples can be distinguished from one another, and how the data from different excitation modes can be best combined to distinguish different species. A support vector machine or a neural network are two well-understood types of machine-learning algorithm that have been found to be effective in this application.

In some embodiments, identifying the one or more substances comprises classifying the one or more substances. Classifying the substances, for example into species or sub-species (or strain), can provide valuable information about the content of the sample.

In some embodiments, identifying the one or more substances does not comprise processing to reduce the contribution to the measured spectra of any photophysical processes in the sample. It is common to perform background subtraction to remove some contributions to the measured light intensity from a sample. However, this "background" can sometimes be dependent on the substance in the sample, and therefore provide valuable additional information to improve classification and detection.

The inelastic scattering of light comprises Raman scattering. Raman scattering is particularly useful due to its dependence on molecular structures that can be used to differentiate different substances.

In some embodiments, identifying the one or more substances does not comprise processing to reduce the contribution to the measured spectra of the fluorescence in the sample. Raman spectroscopy techniques are often designed to remove or minimise fluorescence contributions. However, this "background" can sometimes be dependent on the substance in the sample, and therefore provide valuable additional information to improve classification and detection.

In some embodiments, the polarisation of at least one of the excitation modes comprises linear polarisation, circular polarisation, or elliptical polarisation. These polarisation types are readily achievable with known optical components and techniques.

In some embodiments, two or more of the excitation modes differ from one another in polarisation. The response of substances in the sample can vary significantly depending on the polarisation of light illuminating the sample. Therefore, using different polarisations can emphasise differences between substances in the sample, thereby aiding in their identification.

In some embodiments, the wavelengths of the excitation modes comprise one or more visible light wavelengths and/or one or more infra-red light wavelengths. For example, the one or more visible light wavelengths may comprise a wavelength in the range 400-700nm, and the one or more infra-red light wavelengths may comprise a wavelength in the range 700-3000nm and/or the wavelengths of the excitation modes may comprise one or more of 405nm, 532nm, 633nm, 785nm, and 1064nm. These wavelengths are advantageous for Raman spectroscopy, because they balance a general increase in the magnitude of Raman scattering with decreasing wavelength, with the increased likelihood of degradation of substances in the sample from very short wavelength illumination.

The excitation modes differ from one another in wavelength. Using different wavelengths of excitation modes can change the relative magnitude of Raman peaks, which provides a useful source of information for distinguishing and/or identifying different substances in the sample.

In some embodiments, two or more of the excitation modes differ from one another in wavelength by at least 20nm. Specifying a minimum difference in the wavelengths of excitation modes ensures that the differences between the measured spectra are sufficient to provide additional information to distinguish and/or identify different substances in the sample.

The wavelengths of the excitation modes are such that the measured spectra comprise two or more of a non-resonant Raman spectrum, a pre-resonant Raman spectrum, and a resonant Raman spectrum. Resonant Raman spectroscopy can enhance the magnitude of the observed signal, but increases the non-Raman fluorescence signal from the sample. Using a combination of different Raman regimes can emphasise differences in substance behaviour between regimes, increasing the ability to distinguish substances in the sample.

In some embodiments, illuminating the sample with light of each of a plurality of different excitation modes comprises illuminating the sample simultaneously with light of each of the excitation modes. Simultaneous measurement of the different excitation modes can decrease the time necessary to make a measurement, thereby improving throughput.

In some embodiments, illuminating the sample with light of each of a plurality of different excitation modes comprises illuminating the sample sequentially with light of each of the excitation modes. Sequential measurement of different excitation modes can reduce the complexity of the device required, because a single tuneable or rapidly switchable light source can be used instead of requiring multiple sources for simultaneous excitation.

In some embodiments, the one or more substances comprise microorganisms, for example bacteria or archaea, and identifying the one or more substances comprises classifying the microorganisms. For example, classifying the microorganisms may comprise identifying a category of the microorganisms, wherein the category comprises one or more of a taxonomic group of the microorganisms and a phenotype of the microorganisms. Microorganisms are of particular concern, in view of the need to identify and distinguish antibiotic-resistant sub-species (strains) from other strains. The taxonomic group may comprise a sub-species, strain, species, genus, family, order, or class. The phenotype may comprise a type of anti-microbial resistance or anti-biotic susceptibility.

In some embodiments, the sample comprises microorganisms of two or more categories, and identifying the one or more substances comprises determining a quantity or relative proportion of microorganisms of each of the two or more categories. This can be useful where microorganisms are intentionally present, and a level of different categories of microorganism should be monitored.

In some embodiments, the one or more substances comprise any type of amyloid fibrils, amyloid plaques or its precursors, tau and phospho-tau, huntingtin, or other markers of proteinopathies. In some embodiments, the one or more substances comprise extracellular matrix components, optionally collagen and/or elastin. These substances can be used as early-stage markers of neurodegenerative diseases and dementias such as Alzheimer's disease, Parkinson's disease, Huntington's disease and other proteinopathies.

In some embodiments, the sample is a biological sample. Biological samples are of particular interest for detection of potential disease or contamination in clinical and healthcare settings.

In some embodiments, the sample is obtained from an organism, and the method further comprises determining a type, a likelihood, a severity, and/or a stage of a disease or condition for the organism on the basis of the identification of the one or more substances, optionally wherein the organism is a human. The presence of certain substances in the sample may be indicative of a disease type and stage and so the identification can be used to provide feedback to a subject.

In some embodiments, the sample is obtained from a non-biological source. The method may further comprise determining a likelihood or severity of contamination of the non-biological source on the basis of the identification of the one or more substances. The method is also applicable to analysis of samples in other contexts than samples taken from organisms, in particular for detecting contaminating substances that should not be present in the sample. The sample may be a drinking water, wastewater, or effluent sample. For example, the contamination may be a biological contamination. The method is also applicable to analysis of biological entities including microbial organisms in samples that may be non-biological or environmental such as water based samples mentioned above.

According to a second aspect of the invention, there is provided an apparatus for identifying one or more substances in a sample according to claim 11.

By using multiple different excitation modes to measure the sample, combining the data from all of the excitation modes, and using contributions from plural photophysical processes, the information available for distinguishing between species to be analysed is greatly increased. The method therefore provides improved ability to distinguish different species, while also allowing for faster sample analysis from using an optical technique.

In some embodiments, the illumination source comprises a wavelength-tuneable illumination source. A tuneable source allows measurements to be made at plural wavelengths using only a single source.

In some embodiments, the illumination source comprises a plurality of sub-sources, each sub-source configured to emit light at a different wavelength from the other sub-sources. Having plural sub-sources allow the apparatus to measure excitation modes having multiple different wavelengths simultaneously, allowing for faster measurements.

In some embodiments, the illumination source is configured to emit polarised light. The response of substances in the sample can vary significantly depending on the polarisation of light illuminating the sample. Therefore, using polarised light can emphasise differences between substances in the sample, thereby aiding in their identification.

In some embodiments, the polarisation of the light emitted by the illumination source is tuneable. Tuneable polarisation allows a single source to make measurements at excitation modes having multiple polarisations.

In some embodiments, the illumination source comprises a plurality of sub-sources, each sub-source configured to emit light having a different polarisation from the other sub-sources. Having plural sub-sources allow the apparatus to measure excitation modes having multiple different polarisations simultaneously, allowing for faster measurements.

In some embodiments, the bandwidth of light emitted by the illumination source is sufficiently narrow to resolve a Raman linewidth of 50 cm⁻¹ or less. This resolution allows the apparatus to resolve peaks of sufficient linewidth to distinguish and identify many different substances of interest.

In some embodiments, the detector is configured to detect light reflected and/or backscattered from the sample. A reflection geometry can make the apparatus more compact and reduce the impact of transmitted light from the illumination source that has not interacted with the sample.

In some embodiments, the detector is configured to detect light transmitted through and/or scattered by the sample. A transmission geometry does not require as many reflective optical elements that can affect light from the sample, and can be easier to implement due to fewer constraints on the placement of the detector relative to the illumination source.

In some embodiments, the apparatus further comprises a filtering element configured to remove light at the wavelength of the excitation mode from the light from the sample. For example, the filtering element may comprise a shortpass optical filter, longpass optical filter, notch optical filter, bandpass optical filter, or electro-optic modulator. Raman spectroscopy signals are typically small compared to the intensity of the illuminating source. Filtering out transmitted light at the excitation wavelength makes it easier to discern the signals of interest.

In some embodiments, the apparatus further comprises a detection polariser configured to select light having a predetermined polarisation from the light from the sample. Substances in the sample may affect the polarisation of light, and so using a polariser to measure the light from the sample at different polarisations can provide further information to improve the identification of the substances.

In some embodiments, the apparatus further comprises a resolving element configured to spectrally resolve the light from the sample. For example, the resolving element may comprise a spectrograph, a grating, a prism, or an interferometer. This simplifies the measurement of the measured spectra by resolving the wavelengths of light from the sample.

Embodiments of the invention will now be described, by way of a non-limiting example only, with reference to the accompanying drawings in which corresponding reference symbols indicate corresponding parts, and in which according to some embodiments of the disclosure:
Fig. 1 is a schematic of an apparatus according to an embodiment;
Fig. 2 is a flowchart of a method according to an embodiment;
Figs. 3A to 3F illustrate preparation of a sample for use with the invention;
Figs. 4A to 4C shows classification accuracies of bacterial strains using measured spectra at two different wavelengths individually, and using both measured spectra together;
Figs. 5A to 5C illustrate the identification of fibrils relevant to Alzheimer's disease using principal component analysis;
Figs. 6A and 6B show spectra used to identify bacterial species in a sample using a prior art method with only a single excitation mode;
Figs. 7A and 7B show Raman spectra of S. aureus at 785nm and 532nm excitation respectively;
Figs. 8A and 8B show Raman spectra of P. aeruginosa at 785nm and 532nm excitation respectively;
Figs. 9A and 9B show Raman spectra of hemin and protoporphyrin IX;
Figs. 10A and 10B show Raman spectra of beta-carotene, and xanthophyll;
Figs. 11A to 11C show separation of different bacterial species by principal component analysis using 785nm excitation mode only, 532nm excitation mode only, and a combination of both excitation modes respectively;
Figs. 12A to 12C show classification accuracies using a support vector machine of pure pellets of bacterial strains using 785nm excitation mode only, 532nm excitation mode only, and a combination of both excitation modes respectively;
Figs. 13A to 13C show classification accuracies using a support vector machine of bacterial strains in artificial sputum medium using 785nm excitation mode only, 532nm excitation mode only, and a combination of both excitation modes respectively; and
Fig. 14 shows an illustrative workflow for a possible application of the invention in healthcare and clinical settings.

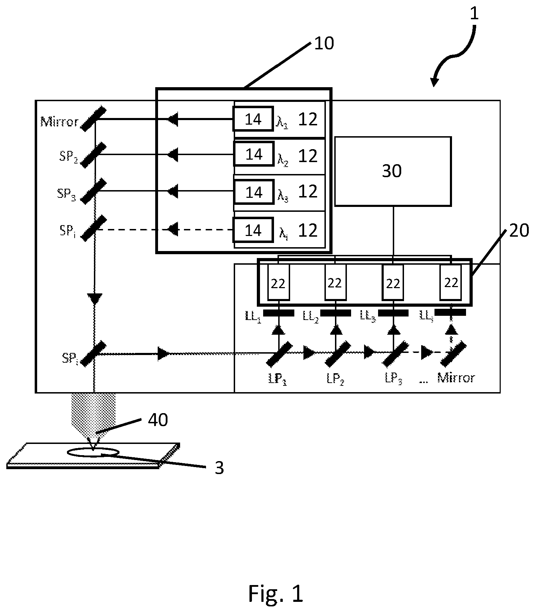

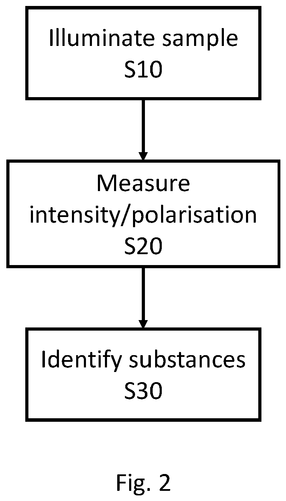

Fig. 1 depicts an apparatus 1 for identifying one or more substances in a sample 3. The apparatus 1 can be used to implement a method of identifying one or more substances in a sample 3, as shown in Fig. 2.

The apparatus 1 comprises an illumination source 10 configured to carry out the step S10 of illuminating the sample 3 with light of each of a plurality of different excitation modes, a detector 20 configured to carry out the step S20 of measuring an intensity and/or polarisation of light from the sample 3 at a plurality of wavelengths to obtain a measured spectrum for each of the excitation modes, and a processing unit 30 configured to carry out the step S30 of identifying one or more substances in the sample 3 using the measured spectra together.

As will be discussed in more detail below, the method uses Raman spectroscopy at multiple wavelengths and/or and multiple polarisation states to obtain additional information about the substances in the sample 3, and incorporates other signals from non-Raman photophysical processes, such as fluorescence, photoluminescence, phosphorescence and scattered light, into the identification of the one or more substances. Unlike existing Raman spectroscopy methods, using multiple excitation modes harnesses the variation expected in Raman scattering and other photophysical signals to extract additional information from the sample 3. The method exploits factors such as appearance and disappearance of resonant peaks, non-resonant peaks, and signals from non-Raman photophysical processes such as fluorescence that are usually ignored as the Raman background.

The apparatus 1 may be referred to as a multi-excitation Raman spectrometer, due to the multiple excitation modes used. The apparatus 1 may be referred to as a Raman spectrometer because of the mode in which it is typically operated, and because Raman scattered signals represent one of the most important components of the light from the sample used for identifying the substances. However, it should be understood, as will be discussed further below, that the apparatus 1 is not limited to detecting only Raman scattering from the sample 3. The measured spectra will include Raman signals, and also components due to other photophysical processes.

The apparatus 1 operates in an epi detection geometry, where the light from the illumination source 10 and the light from the sample 3 both pass through the same focusing lens 40 on their way to and from the sample 3 respectively. In other words, the detector 20 is configured to detect light reflected and/or backscattered from the sample 3. However, it is not essential that the apparatus 1 use this type of detection geometry. For example, the apparatus 1 may use a geometry where light from the illumination source 10 and light from the sample 3 pass through different lenses on their way to and from the sample 3 respectively. This could be achieved by capturing the light from the sample 3 at an angle to the light from the illumination source 10, such as 45° or 90°. In other embodiments, the apparatus 1 may use a transmission detection geometry, where the detector 20 is configured to detect light transmitted through and/or scattered by the sample 3. In this case, light scattered by the sample 3 is light scattered by an angle too small to be considered backscattering, e.g. less than 90°. In the transmission geometry, a second lens on the underside of the sample 3 would collect the light and direct the captured light to the detector 30. In any detection geometry, the detector 20 will also detect light emitted from the sample 3, for example by fluorescence or similar processes.

Light from the illumination source 10 is directed down to a common focusing lens 40 and illuminates the sample 3. Light from the sample is then collected by the focusing lens 40. The light from the sample 3 is red-shifted relative to the light from the illumination source 10, and therefore can be directed towards the detector 20 pathway via a dichroic mirror, in this case a short-pass dichroic mirror SPᵢ. The detected light is then combined and processed by the processing unit 30.

The method comprises illuminating S10 the sample 3 with light of each of a plurality of different excitation modes. This is achieved using the illumination source 10. The light is used to excite molecules in the sample 3 to probe their optical properties. This approach allows the method to generate new information (different peak intensities) at the same variables (Raman shift) due to variation in the Raman cross-sections of different vibrations as a function of the excitation mode.

The illumination source 10 is typically a laser, which can provide high intensity and narrow bandwidth. Preferably, the bandwidth of light emitted by the illumination source 10 is sufficiently narrow to resolve a Raman linewidth of 50 cm⁻¹ or less, preferably 25cm⁻¹ or less, more preferably 10cm⁻¹ or less. The power of the illumination source 10 (e.g. laser) is set as required by the particular application, depending on factors such as the detection geometry. The power may vary between excitation modes if appropriate.

Light from the illumination source 10 is routed through appropriate optics to the sample 3. For example, the light from the illumination source 10 can be delivered through a microscope, through an optical fibre, or directly onto the sample 3. After interaction with the sample 3, light from the sample 3 is collected and directed towards the detector 20.

In Fig. 1, the illumination source 10 comprises a plurality of sub-sources 12. Each sub-source 12 is configured to emit light at a different wavelength from the other sub-sources 12. For example, the sub-sources 12 may comprise different lasers emitting light of different wavelengths. Preferably, the bandwidth of light emitted by each sub-source is sufficiently narrow to resolve a Raman linewidth of 50 cm⁻¹ or less, preferably 25cm⁻¹ or less, more preferably 10cm⁻¹ or less. Alternatively or additionally, a plurality of the sub-sources 12 may be provided by a single laser whose beam is split and the split beams modified by appropriate optics to shift their frequencies, with each sub-source 12 corresponding to one of the split beams. Alternatively or additionally, the illumination source 10 may comprise a wavelength-tuneable illumination source 10, such as a tuneable laser source.

Illuminating S10 the sample 3 with light of each of a plurality of different excitation modes comprises illuminating the sample 3 sequentially with light of each of the excitation modes. This embodiment may be used where a single wavelength-tuneable illumination source 10 is used, and the wavelength of the illumination source 10 is shifted between excitation modes having different wavelengths. This has the advantage that only a single illumination source 10 is used, which can reduce the cost and complexity of the apparatus 1.

Alternatively, illuminating S10 the sample 3 may comprise illuminating the sample 3 simultaneously with light of each of the excitation modes. This may be performed in embodiments where the illumination source 10 comprises a plurality of sub-sources 12. This may have the advantage of increased speed of processing, because plural excitation modes can be measured in a shorter time.

In some embodiments, simultaneous and sequential illumination of the sample 3 may also be combined. For example, illuminating S10 the sample 3 with light of each of a plurality of different excitation modes may comprise illuminating the sample 3 sequentially with light of two or more subsets of the excitation modes, wherein the light of the excitation modes in each subset of excitation modes is applied simultaneously. This may be advantageous where the number of excitation modes desired exceeds the number of sub-sources 12 it is practical to include in the apparatus 1, but it is still desirable to reduce processing time.

The plural excitation modes differ in one or both of wavelength and polarisation. This allows the different response of the sample 3 to different wavelengths and/or polarisations of light to be used together to improve identification of the substances in the sample 3.

The number of excitation modes to be used, and the wavelengths and/or polarisations of each excitation mode, will depend on the application. The number of excitation modes, and the choice of wavelength and/or polarisation for each excitation mode, may be pre-set in a particular apparatus 1 intended for a particular application. For example, the choice may depend on the substances that are to be detected in the sample 3. In some embodiments, the number of excitation modes, and the wavelength and/or polarisation of each excitation mode, may be selectable by a user. This may be advantageous for research applications, or where the apparatus 1 may be used for identifying a wide range of different substances.

The wavelengths of the excitation modes may comprise one or more visible light wavelengths and/or one or more infra-red light wavelengths. The one or more visible light wavelengths may comprise a wavelength in the range 400-700nm. The one or more infra-red wavelengths may be any type of infra-red, for example a near infrared wavelength (typically considered to cover the range of approximately 700-1400nm), a shortwave infrared wavelength (typically considered to cover the range of approximately 1400-3000nm), a mid-infrared wavelength, or a far-infrared wavelength. In some embodiments, the infra-red light wavelength may comprise a near infrared wavelength or a shortwave infrared wavelength, i.e. a wavelength in the range 700-3000nm. Using these different wavelengths allows the apparatus 1 to acquire resonant, pre-resonant, and non-resonant Raman spectra as will be discussed further below. In some embodiments, the wavelengths of the excitation modes comprise one or more of 405nm, 532nm, 633nm, 785nm, and 1064nm.

Existing publications on 2D Raman spectroscopy, where variation in the excitation wavelength is applied, exclusively use excitation wavelengths in the ultra-violet (UV) or deep ultra-violet (DUV) range where the excitation wavelength is around 200-300 nm. This wavelength range is used to exploit the resonant and pre-resonant enhancement of Raman signal achievable in this range. In contrast to this, the present method preferably uses excitation wavelengths across the optical and NIR range. This means the present method does not require the use of resonance and pre-resonance conditions to be able to obtain results, unlike in previous methods (e.g. [36]). Further, the present method can use more readily available and affordable lasers in the visible and near-infrared ranges for the illumination source 10. The use of visible and IR wavelengths also allows the present method to be used on biological samples.

The specific examples for which results are shown below use 405nm, 532nm and 785nm lasers for the illumination source 10. However the present method is extensible to any number of different excitation modes having any wavelength suitable for the analysis of different chemical species and different types of samples. It is the combination of multiple measured spectra used together in the identification that provides improved classification accuracy in the present method. Excitation modes having a wavelength in the UV may be used if the specific application was likely to benefit from its inclusion, for example for the analysis of dipicolinic acid in spore detection.

In some embodiments, two or more of the excitation modes differ from one another in wavelength. The intensity of scattered light has a strong dependence on the wavelength of the excitation source: $P ∝ \frac{1}{{λ}^{4}}$ Changing the wavelength of the excitation modes will change the response of individual Raman vibrational modes in the substances in the sample 3. The response of individual vibrational modes to the different wavelengths of the excitation modes causes distinctive changes to the peak heights in the Raman spectra measured by the detector 20. The present method takes advantage of these differences to provide further information about the substances in the sample 3. This effectively adds a new dimension to the measured spectra (excitation wavelength) different from the usual single dimension of Raman shift in conventional Raman spectra.

The two or more of the excitation modes may differ from one another in wavelength by at least 20nm, preferably at least 25nm, more preferably at least 50nm, most preferably at least 100nm. A minimum difference between the wavelengths ensures that the differences between the measured spectra are sufficient to provide useful additional information for identifying the substances.

Raman cross-sections of energy levels in molecules (e.g. due to chemical bonds) vary with illumination wavelength. The Raman cross-sections become enormously enhanced as the illumination wavelength approaches and finally reaches, an electronic transition. This enhancement is known as resonant or pre-resonant enhancement. The enhancement of peaks based on excitation wavelength is due the resonance or pre-resonance affect, which was first shown by the Albrecht A-term pre-resonance approximation equation for totally symmetric transitions. ${σ}_{Raman} = {Kω}_{Pu} {\left({ω}_{Pu}-{ω}_{vib}\right)}^{3} {\left(\frac{\left({{ω}_{Pu}}^{2}+{{ω}_{0}}^{2}\right)}{{\left({{ω}_{0}}^{2}-{{ω}_{Pu}}^{2}\right)}^{2}}\right)}^{2}$ where K is a collection of frequency independent factors of Raman molecules, ω₀ is the frequency of the molecular absorption peak and ω_{vib} is the vibrational transition energy.

Conventionally Raman spectroscopy is carried out under non-resonant excitation conditions, that is, in the absence of electronic transitions when the illuminating light is much lower in energy than the absorption peak (ω₀).

However, use of resonance excitation in Raman spectroscopy is well known to enhance signals. The equation makes it clear that when the pump wavelength is near to, or at, the absorption maximum the Raman cross-section increases, and therefore the signal increases, giving the pre-resonance or the resonant effect, respectively.

Many organic molecules, and molecules with small molecular weights, have absorption in the ultra-violet spectral region. Excitation wavelengths in the DUV may also take advantage of self-absorption and laser penetration depth. Resonance Raman excitation in the UV and DUV regions has been used to improve the identification of explosive materials [11]. Pre-resonant enhancements can also provide large boosts in the intensity of certain vibrational modes within a Raman spectrum [12]. The use of resonant Raman spectroscopy has also been applied to the study of cytochrome *cd₁* from bacteria [13], and UV resonance has been applied to whole bacteria and endospore biomarkers [14].

However, while these techniques have been used to enhance Raman signals using resonance, little attention has been paid to the differences between resonant, pre-resonant, and non-resonant behaviour. In some embodiments of the present method, the wavelengths of the excitation modes may be such that the measured spectra comprise two or more of a non-resonant Raman spectrum, a pre-resonant Raman spectrum, and a resonant Raman spectrum. Using the different behaviours under non-resonant, pre-resonant, and resonant excitation, along with other effects such as intrinsically fluorescent or luminescent molecular components in the samples, can enhance the ability of the method to distinguish between substances in the sample 3.

It has further been found that utilising and observing the polarisation properties of Raman signals together with the use of multiple excitation modes, which may have differing wavelengths, provides another way to increase the information available to improve accuracy of identification of substances in the sample 3. While the polarisation dependence of Raman spectra is known, this information has not been fully exploited for identifying substances or for diagnostics, and the combination with multiple excitation wavelengths has not previously been attempted.

In some embodiments, the illumination source 10 is configured to emit polarised light. In some embodiments two or more of the excitation modes differ from one another in polarisation. The response of vibrational modes in substances in the sample 3 to a change in the polarisation state of the illumination source 10 provides further information about the substance. As for wavelength above, this effectively adds a new dimension to the measured spectrum (polarisation state of illumination) different from the usual single dimension of Raman shift in conventional Raman spectra, thereby further increasing the ability of the method to differentiate different substances in the sample 3.

As shown in Fig. 1, the illumination source 10 comprises emission polarisers 14 to polarise the light from the sub-sources 12. In the example of Fig. 1, a separate emission polariser 14 is provided as part of each of the sub-sources 12. Therefore, in this embodiment, where the illumination source 10 comprises a plurality of sub-sources 12, each sub-source 12 may be configured to emit light having a different polarisation from the other sub-sources 12, according to the setting of each emission polariser 14. However, it is not essential that each sub-source 12 is configured to emit light having a different polarisation from every other sub-source 12. For example, a first subset of the sub-sources 12 may be configured to emit light having a first polarisation, and a second subset of the sub-sources may be configured to emit light having a second polarisation, and so on for further subsets where this is desirable.

In other embodiments, for example where the illumination source 10 does not comprise plural sub-sources 12 or where the excitation modes are not required to differ in polarisation, only a single emission polariser 14 may be provided. The use of an emission polariser 14 is not required, and in still other embodiments, no emission polariser 14 may be used at all, such that light reaching the sample 3 is unpolarised.

The polarisation of the light emitted by the illumination source 10 may be tuneable. For example, the emission polarisers 14 may be configured to be rotatable or otherwise adjustable to change the polarisation of light reaching the sample 3 from the illumination source 10. The polarisation of at least one of the excitation modes may comprise any suitable type of polarisation, for example linear polarisation, circular polarisation, or elliptical polarisation.

The apparatus 1 further comprises a detector 20 configured to carry out the step S20 of measuring an intensity and/or polarisation of light from the sample 3 at a plurality of wavelengths to obtain a measured spectrum for each of the excitation modes. Any suitable light detector may be used, for example a spectrometer, camera, CCD, CMOS detector, photomultiplier tube (PMT) that collect a single wavenumber at a time and are exposed to the full spectral range of interest in steps, or any other detector appropriate to the application. The detector 20 preferably has sufficient wavelength-discrimination capabilities to acquire high-quality Raman spectra at high speeds, while also collecting the information from contributions to the measured spectra due to the other photophysical processes. Where applicable, the detector 20 may resolve the light from the sample 3 for measurement at the plurality of wavelengths using a resolving element configured to spectrally resolve the light from the sample 3. The resolving element may comprise any suitable element such as a spectrograph, a grating, a prism, or an interferometer.

The plurality of wavelengths would cover a range of wavelengths relative to the wavelength of the corresponding excitation mode, the range being large enough to detect any expected shift due to Raman or other inelastic scattering. The range may be above, below, or otherwise around the wavelength of the corresponding excitation mode. The range may be expressed in terms of wavenumber, rather than wavelength, for example a range of at least 500cm⁻¹, preferably a range of at least 1000cm⁻¹, more preferably at least 2000cm⁻¹, most preferably at least 3000cm⁻¹.

As discussed above, the apparatus 1 may be arranged to direct light from the sample 3 to the detector 20 in any suitable manner depending on the optical arrangement that is most suitable for the particular application. For example, the light from the sample 3 may be collected through a microscope, an optical fibre, or directly from the sample.

In the example of Fig. 1, an array of long-pass filters in the form of long-pass dichroic mirrors LPi directs the light from the sample 3 to the detector 20. In the example of Fig. 1, each sub-source 12 is configured to emit light of a different wavelength, and the detector 20 comprises a plurality of sub-detectors 22, each sub-detector being appropriate for detection of one of the different wavelengths or wavelength sub-ranges emitted by the sub-sources 12. Each sub-detector 22 may be sensitive to a range of wavelengths. The exact range will depend on the type of detector chosen and the arrangement of the optical setup. The exact range of wavelengths to which the detector is sensitive is not critical, as long as it is possible to distinguish the signal due to the light from the different excitation modes based on the outputs of the sub-detectors 22. For example, in Fig.1, the long-pass dichroic mirrors LPᵢ are configured to direct the appropriate component of the light from the sample 3 to an appropriate sub-detector 22 depending on the wavelength. This means that each sub-detector 22 may be sensitive to relatively broad, or even overlapping, ranges of wavelengths, because the other optical components ensure that the light reaching each sub-detector 22 is already separated by wavelength. In other embodiments, each sub-detector 22 may be sensitive to a relatively narrow range of wavelengths around the wavelength emitted by the corresponding sub-source 12. In this case, it may not be necessary to filter or separate the light from the sample before it is directed to the sub-detectors 22. Each sub-detector 22 may comprise a resolving element as appropriate.

This arrangement of sub-detectors 22 and filters is advantageous when the apparatus 1 is configured to illuminating the sample 3 simultaneously with light of each of plural excitation modes, because the detector 20 can measure the light from the sample 3 for each excitation mode simultaneously. However, in other embodiments, the detector may not comprise plural sub-detectors 22, and may comprise only a single sub-detector 22. In this case, each excitation mode may be measured sequentially by the detector 20.

The apparatus 1 may further comprise a filtering element LL configured to remove light at the wavelength of the excitation mode from the light from the sample 3. This means that measuring an intensity and/or polarisation of light from the sample 3 for each of the excitation modes comprises filtering out light at the wavelength of the excitation mode. This is advantageous because it can remove reflected or transmitted light from the illumination source 10 that has not significantly interacted with the sample 3. This light does not provide as much information about substances in the sample 3, and may drown out other signals of greater interest.

In Fig. 1, the apparatus 1 comprises plural filtering elements LLᵢ, one for each of the sub-detectors 22. The filtering elements LL comprise laser line filters, but may comprise any other suitable filter such as a shortpass optical filter, longpass optical filter, notch optical filter, bandpass optical filter, or electro-optical modulator. The filtering elements LL preferably filter a band of wavelengths that is wide enough to block most of the bandwidth of light from the illumination source 10 at the corresponding excitation mode, but narrow enough not to block light that has been Raman-shifted by substances in the sample 3.

The interaction of the light from the illumination source 10 with the sample 3 may affect or change the polarisation. This change in polarisation can provide additional information about substances in the sample 3. Therefore, the detector 20 may be configured to measure the polarisation of light from the sample 3 for each excitation mode at the plurality of wavelengths, as well as the intensity. The apparatus 1 may comprise a detection polariser configured to select light having a predetermined polarisation from the light from the sample 3. A detection polariser may be provided for each sub-detector 22. The detection polariser may comprise an automated or manual insertion of polarisation optics in the beam paths from the sample 3 to the detector 20. The detection polariser is preferably tuneable, such that any change in polarisation relative to the light emitted by the illumination source 10 can be detected as part of the measured spectra.

The apparatus 1 further comprises a processing unit 30 configured to carry out the step S30 of identifying one or more substances in the sample using the measured spectra together. The present method further differs from conventional Raman spectroscopy in how the measured spectra are analysed to identify or classify substances in the sample 3.

Conventionally, Raman spectra are analysed to identify sets of peaks that correspond to particular substances, depending on the vibrational signatures of molecules or materials making up the substance. Raman spectra, are also often regarded as invariant to the light used for illumination of the sample 3. Although the Raman scattered wavelengths shift with the illumination wavelength, the difference in energy between the illuminating and scattered wavelengths remains constant even with different illumination wavelengths. However, the invariance is only of the position (i.e. wavelength shift relative to the illumination wavelength) of the peaks. The intensities of peaks and of the background can vary, because the intensities depend on the polarizability, susceptibility, and other properties of the substances, which is in turn dependent on wavelength. Hence, the inventors have found that the intensities at each wavelength, and in particular the differential intensities between peaks, provide additional information that can be helpful in identifying substances in the sample 3. The polarisation of the light from the sample 3, in particular of the Raman signal, is also dependent on the wavelength of the light used to illuminate the sample. Hence, differential polarisation properties at different wavelengths in the measured spectra also provide an additional source of information.

The present method uses the measured spectra together in the identification. The present method takes advantage of the physical principle that Raman cross-sections (strength of Raman scattering interactions) vary with the wavelength and polarisation of the illuminating light. Hence, there are variations in peak intensity ratios between the measured spectra corresponding to different excitation modes that arise as a result of the differences in wavelength and/or polarisation of the light from the illumination source 10 between excitation modes.

One straightforward embodiment of using the measured spectra together, used in the examples for which results are given below, is the following. The measured spectra for each excitation mode are collated together, for example by concatenating the measured spectra into a single list of values. In this case, each individual measurement or 'observation' (corresponding to a single measurement of the intensity or polarisation of light from the sample at one of the plurality of wavelengths for one of the excitation modes) is represented by an integer, and considered as an independent variable. Therefore, the first measured spectrum for the first excitation mode composed of j observations (one for each intensity or polarisation at each of the plurality of wavelengths) will be entries I₁ to Iⱼ in the single list. The second measured spectrum for the second excitation mode will be entries Iⱼ₊₁ to Iₖ, and so on until all the observations in the final measured spectrum are numbered. After all measured spectra are concatenated as above, numerical analysis is performed on the single combined list of values. Of course, the method is not limited to this method of combining the measured spectra for their use together in the identification, and any other suitable method may be used, for example representing the plurality of measured spectra as a two-dimensional array of values.

In some embodiments, identifying the one or more substances comprises using a multivariate analysis. This analysis method explicitly recognises the multidimensionality of the measured data and the information contained therein. The multivariate analysis may comprise any suitable technique, for example principal component analysis or linear discriminant analysis. An implementation using principal component analysis is demonstrated further below.

Where identifying the one or more substances comprises using a multivariate analysis, identifying the one or more substances may comprise combining the measured spectra using the multivariate analysis to obtain a multi-dimensional signature of the sample 3. For example, the multi-dimensional signature may combine the measured spectra corresponding to different excitation modes using varying weights and/or applying various processing algorithms to obtain a multi-dimensional signature. These weights and algorithms may be determined based on training data from measurements of known samples to best highlight differences between substances of interest. The combining of the measured spectra may differ depending on the particular application.

Identifying the one or more substances may then further comprise comparing the multi-dimensional signature to one or more reference signatures. The reference signatures may be determined using training data from measurements of known samples containing known substances of interest.

In some embodiments, identifying the one or more substances comprises using a machine-learning algorithm. The machine-learning algorithm may comprise any suitable machine-learning algorithm, for example a support vector machine (SVM) or a neural network. This allows for the combination of data from the plural measured spectra for increased accuracy and precision in the identification of substances in the sample 3. An implementation using a SVM for detection and strain-level identification of common bacterial respiratory pathogens in complex media is demonstrated further below. As for the multivariate analysis embodiments, the machine-learning algorithm may be trained on training data from measurements of known samples.

These techniques allow for unsupervised or supervised classification for a completely unknown sample 3. Identification of substances in the sample 3 can be performed against a trained model obtained using data measured for samples of known substances of interest, for example bacteria, explosives, substances of abuse, etc. The result of this identification can be used for classification or identification of the substances, or diagnosis of a subject.

Identifying the one or more substances may comprises classifying the one or more substances. For example, the identifying may comprise classifying bacteria in the sample 3 into a particular species and/or strain. In other cases, the identifying may comprise classifying a substance in the sample 3 into a particular class of compounds.

The identifying of the one or more substances uses contributions to the measured spectra from a plurality of photophysical processes in the sample 3 including inelastic scattering of light. As mentioned above, Raman scattering of light is one of the most important contributions to the measured spectra, and so the inelastic scattering of light may comprise Raman scattering. However, the present method makes use of contributions from other photophysical processes as well. This is in contrast to existing methods that may measure Raman signals using light of plural different wavelengths.

In existing methods, such as shifted-excitation Raman difference spectroscopy (SERDS), the signals measured using illumination at different wavelengths are generally used for background subtraction. For example, measurements at two very similar wavelengths may be subtracted from one another to reject fluorescence, which has a weaker dependence of intensity on small shifts in wavelength than Raman signals. Therefore, in such existing methods, the measurements at different wavelengths of illuminating light are actually used to remove contributions from other photophysical processes, rather than to include them as additional sources of information. The identification of one or more substances may therefore not comprise processing to reduce the contribution to the measured spectra of any photophysical processes in the sample.

In other methods, lengthy photo-bleaching steps are required to reduce the fluorescence in the samples [15]. Rusciano *et* al used single-excitation mode Raman spectroscopy to detect pathogens in CF patient samples. However, their method required 15 minutes of pre-bleaching before spectral features began to appear, and 30 minutes of photo-bleaching before the spectral features became sufficiently prominent to achieve a comparable level of accuracy (<95%). In contrast, the present method can yield high-quality measured spectra and corresponding identification results with exposure times of ~1 minute.

One potential source of further information is contributions to the measured spectra from fluorescence in the sample 3, and the plurality of photophysical processes in the sample 3 used by the identifying step S30 may further include fluorescence. In this case, identifying the one or more substances may not comprise processing to reduce the contribution to the measured spectra of the fluorescence in the sample. In contrast to the contributions from Raman scattering, fluorescence (or luminescence) relies on electronic transitions where the emission intensity is dependent on the illuminating wavelength, but the wavelength of emitted light is invariant within a band of excitation wavelengths. The contributions to the measured spectra from fluorescence are less dependent on the wavelength of illuminating light (within a range) than the contributions from Raman scattering, as long as the illuminating light is able to excite an electronic transition. However, both Raman scattering and fluorescent intensities depend on the polarisation state of the illuminating light. Fluorescent signals are highest when the transitional dipole of the chromophore is aligned with the polarisation of the light from the illumination source 10.

However, the method may also make use of any other light that is emitted or scattered from the sample 3 as a result of any other photophysical process. The plurality of photophysical processes in the sample may further includes one or more of fluorescence, photoluminescence, phosphorescence. The plurality of photophysical processes may also include inelastic scattering processes other than Raman scattering, for example Brillouin scattering. The plurality of photophysical processes may also include elastic scattering, such as Rayleigh or Mie scattering, and reflection. Where contributions from elastic scattering processes are included, the apparatus 1 should not include filtering elements LL that remove light at the wavelength of the excitation mode from the light from the sample 3. Alternatively, the measurement of elastic scattering contributions may be made using a separate apparatus or measurement process, and the resulting measurements combined with the measured spectra by the processing unit 30.

The combined utilisation of excitation modes differing in both wavelength and polarisation is particularly advantageous due to the above-mentioned differences in how different photophysical processes respond to changes in wavelength and polarisation. This more holistic utilisation of combined Raman and fluorescence spectra obtained at multiple wavelengths (without or with polarised detection) provides improved precision and accuracy in identifying substances in a sample 3.

The method allows for label-free and non-invasive detection of substances in the sample 3. The method further has increased distinction capability compared to conventional Raman spectral analysis.

In some embodiments, the sample may be a biological sample, i.e. a sample obtained from a living organism such as a human or animal. The method may be used for detection of microbial pathogens. The one or more substances may comprise microorganisms, for example bacteria or archaea, and identifying the one or more substances may comprise classifying the microorganisms. Classifying the microorganisms may comprise identifying a category of the microorganisms. Categories could be defined in various ways depending on the intended applications. For example, the category may comprises a taxonomic group of the microorganisms. The taxonomic group may comprise a sub-species, strain, species, genus, family, order, or class. The increased distinction capability of the present method is highly desirable in cases where the differences in conventional Raman spectra are less prominent, e.g. differentiating between different strains of the same bacterial species.

The category may additionally or alternatively comprise a phenotype of the microorganisms. The method can also be used, for example, for detection of anti-microbial resistance, where the phenotype comprises a type of anti-microbial resistance or anti-biotic susceptibility.

Conventional Raman spectroscopy, and its resonantly-enhanced variant, have been used in the analysis of bacterial and biological samples. However, previous methods are predominantly used to detect the presence of a single biomolecule, and are restricted to using a single wavelength of illuminating light. The present method is not limited thereto, and in some applications the sample may comprise microorganisms of two or more categories. Identifying the one or more substances may comprise determining a quantity or relative proportion of microorganisms of each of the two or more categories.

Additionally, the present method is also suitable for applications in disease areas other than microbial infection. For example, the method may be applied to detect neurodegeneration, identify types and/or sub-types of neurodegenerative disease, and/or determine a stage of disease. Examples of neurodegenerative diseases for which the method may be suitable include Alzheimer's disease, Parkinson's disease, Huntington's disease and other proteinopathies. The one or more substances may comprise any type of amyloid fibrils, amyloid plaques or its precursors, tau and phospho-tau, huntingtin, or other markers of proteinopathies. The one or more substances may comprise neurofilament-light chain, which reflects axonal damage and can be found in cerebrospinal fluid and/or blood plasma. The one or more substances may comprise extracellular matrix components, optionally collagen and/or elastin.

The present method can be used with unprocessed clinical samples directly, and does not require the use of nanoscale material additives for enhancing signals, such as in surface-enhanced Raman spectroscopy (SERS). However, the method is still compatible with the use of such additives and so can be applied to SERS too. The present method solves a key problem in clinical diagnostics, which is the need for a rapid assay for determining the identity of contaminant material or causative agents of disease in samples of complex composition. The present method can provide rapid data to inform diagnosis, classification, and monitoring of disease, health, or treatment status, for example by a medical professional. The method can also allow for categorising, classification, or stratification of patients or sample or patient cohorts.

Where the sample 3 is obtained from an organism, such as a human, the method may further comprise determining a type, a likelihood, a stage, and/or a severity of a disease or condition for the organism on the basis of the identification of the one or more substances.

The sample 3 may alternatively be obtained from a non-biological source or may be of non-biological or environmental origin. The present method can be used to solve a key problem in quality assurance across a variety of industries, which is the need for a rapid assay for determining the identity of contaminant material in samples of complex composition. These industries may include sectors such as homeland security and the military, the petrochemical and food industries, or any other quality control applications that may be concerned with microbial or non-biological contamination.

Presence of bacteria from industrial processes can cause damage to the local ecology, for example high nutrient loads can cause algal blooms. Monitoring bacteria within an industrial plant can help minimise the nutrient load entering the environment. This discourages and prevents out of control growth of bacteria, algae, etc.

The method can be used to monitor municipal drinking water and/or wastewater treatment, either inside a plant or outside of a plant, for example for monitoring bacteria content of effluents entering the natural environment. The method can also be used to measure or analyse industrial runoffs and effluents, for example from pharmaceutical plants, abattoirs, or manufacturing processes that use microbes such as cheese making, paper pulping, etc.

Examples of possible origins of the sample 3 when obtained from a non-biological source include a water source (such as tap water, a water supply plant, a river, sea, or other natural body of water). The sample 3 may be obtained from drinking water, wastewater, contaminated water, water-based runoffs, domestic or industrial effluents. The sample 3 may be obtained from an industrial water process, for example any part of a wastewater treatment system from sewer influent to effluent into a river, sea, or other natural body of water.

In some applications, the sample 3 may be obtained from low-oxygen liquids, waters, sludges (thickened waste material containing bacteria, other microorganisms, organic matter either before or after breakdown, etc.), and/or slurries. This may enable monitoring and/or measurement of anaerobic microorganisms such as anaerobic bacteria.

The method may further comprise determining a likelihood or severity of contamination of the non-biological source on the basis of the identification of the one or more substances. The contamination of the non-biological source may be a biological contamination.

Raman spectrometers can be highly miniaturised. Laser-equipped Raman spectrometers are commercially available that are of small form factor (<10cm x 6cm). Such devices could be used to implement an apparatus for carrying out the present method, and allow the method to be performed on-site by staff with minimal training. For example, diagnostic tests could be performed in the ward or in less resource-rich operator-independent settings. Although the results below are presented on a stationary sample 3, the method is also applicable to analysis of flowing liquids, for example in channels and microfluidic devices.

An experiment was carried out to demonstrate the application of the method to identifying substances in artificial sputum medium (ASM).

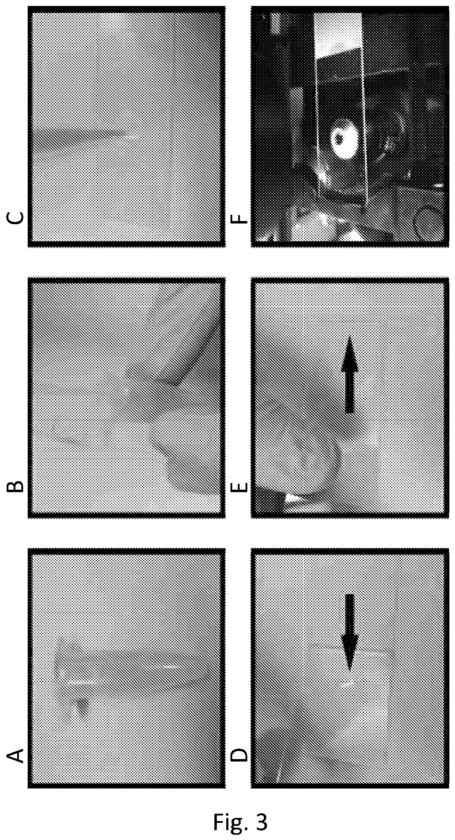

Fig. 3 shows sample preparation, as used to make samples of infected sputum ready for measurement. In Fig. 3A, infected sputum is collected from a patient. In Figs. 3B & 3C, a 10µL aliquot of the sample is taken and deposited onto a clean quartz microscope slide. In Figs. 3D & 3E, a second clean slide (spreader slide) is drawn back towards the droplet at a 30-40 degree angle, ensuring even contact with the lower slide until the droplet attaches to the spreader slide. The spreader slide is pushed back away from the droplet in a smooth motion, drawing the droplet out across the quartz slide in a thin smear. In Fig. 3F, the smeared slide is mounted in the apparatus, ready for acquisition of the measured spectra, as discussed above.

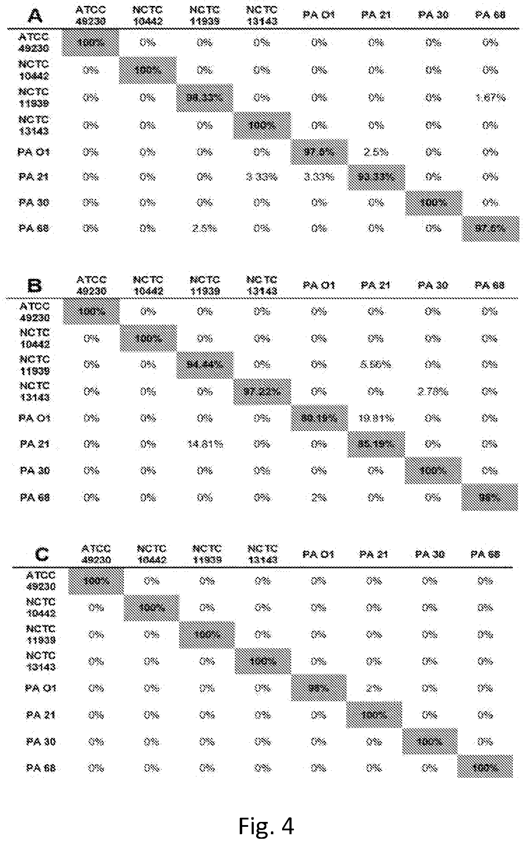

Measured spectra were obtained for samples of different bacterial strains in artificial sputum media for two excitation modes having wavelengths of 785nm and 532nm. Fig. 4 shows tables of percentage classification accuracies using a SVM to analyse the measured spectra. Correct classifications are shown in the highlighted boxes for each strain, with numbers in the remaining boxes indicating the percentage of samples for that species that were incorrectly identified as another strain. Perfect classification would be represented by scores of 100% across all the highlighted boxes, with scores of 0% in all other boxes.

Fig. 4A shows the classification accuracies using only the measured spectrum for the 785nm excitation mode, and Fig. 4B shows the classification accuracies using only the measured spectrum for the 532nm excitation mode. Fig. 4C shows the classification accuracies using both of the measured spectra concatenated and analysed together according to the present method. Fig. 4 demonstrates that the classification accuracies were higher using the present method than the conventional analysis only using a single excitation mode.

Table 1 below shows a summary of results for another example application of the present method. In this example, excitation modes were used that varied in both wavelength and polarisation to distinguish different bacterial species and strains. In this example, principal component analysis (PCA) was used for unsupervised classification of the measured spectra into distinct groups. The results in Table 1 show that classification of bacterial samples using excitation modes that differ in polarisation was more effective than using depolarised Raman spectra or non-polarisation resolved Raman spectra alone. As for Fig. 4, the results are shown for classification based on only single wavelength data (405nm or 532nm), or data from both wavelengths concatenated and used together (405+532).

**Table 1**

| Spectral Combination | | Classifier Accuracy (%) |
|---|---|---|
| Wavelength (nm) | Polarisation | PCA |
| 405 | Regular | 95.0 |
| | Depolarised ( ⊥ / ∥) | 96.7 |
| | Regular & Depolarised | 97.5 |
| 532 | Regular | 93.3 |
| | Depolarised ( ⊥ / ∥) | 100.0 |
| | Regular & Depolarised | 100.0 |
| 405+532 | Regular | 77.5 |
| | Depolarised ( ⊥ / ∥) | 81.7 |
| | Regular & Depolarised | 100.0 |

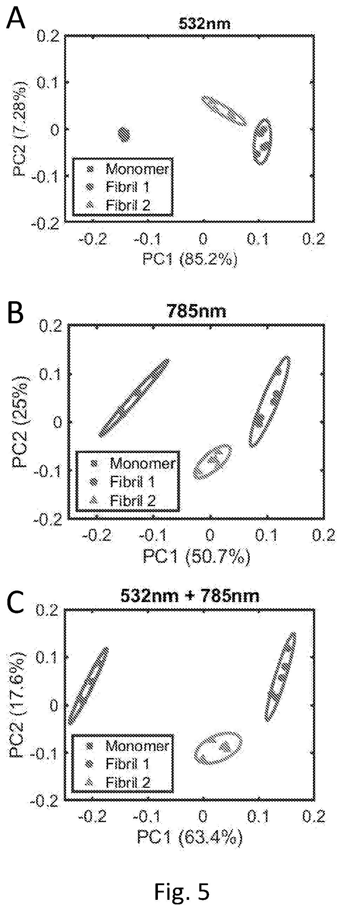

As mentioned above, the present method can be used for other applications such as identifying neurodegenerative disease. Fig. 5 shows the results of PCA applied to measured spectra from samples including fibrils relevant to Alzheimer's disease. In Alzheimer's disease the tau protein forms aggregates of fibrils in the brain but type of fibrils and their nature indicates the nature and maturity level and thus the type/sub-type and progression of disease.

In Fig. 5, PCA analysis results of Raman spectra acquired with 532 nm and 785 nm excitation are presented. The PCA scores plots are shown of measured spectra acquired with (A) 532 nm only, (B) 785 nm only, and (C) 532 nm+785 nm combined according to the present method. The separation between clusters improves in (C) where the measured spectra are used together according to the present method. In other words, the classification becomes clearer after combining the data from multiple excitation modes having different wavelengths.

In a further experiment, the present method is used to detect and characterise the respiratory pathogens Pseudomonas aeruginosa and Staphylococcus aureus. Staphylococcus aureus and Pseudomonas aeruginosa are Gram-positive and Gram-negative representatives of the primary pathogens present in cystic fibrosis in children and adults, respectively. Planktonic specimens were analysed both in isolation and in artificial sputum media. The resonance Raman components, excited at different wavelengths, were characterised as carotenoids and porphyrins.

The bacteria used were S. aureus (SA) strains NCTC 10442, NCTC 11939, NCTC 13143 (EMRSA-16), and ATCC 49230, and P. aeruginosa (PA) strains PAO1, PA21, PA30, and PA68 (REC No: 08/H0502/126). Staphylococcus epidermidis ATCC 35984 and Pseudomonas fluorescens NCTC 13525 were species used for genus differentiation analyses. All bacterial strains were grown in LB broth (Formedium, UK) for 18 hours at 37°C, with shaking at 130 rpm. Staphylococcus species were cultured with aeration. Bacterial cultures were washed twice in water by centrifugation at 4000 g for 10 minutes in a Heraeus Megafuge centrifuge. The resulting pellet was applied to a fused quartz microscope slide (UQG Optics, UK) and dried by gently heating, for subsequent spectroscopic analysis. Artificial sputum media (ASM) was prepared by adapting the methodology described by Sriramulu (mucin from pig stomach mucosa 5g/l, salmon sperm DNA 4g/l, diethylene triamine pentaacetic acid 5.9mg/l, NaCl 5g/l, KCl 2.2g/l, Tris Base 1.81g/l, egg yolk emulsion 5ml/l, casamino acids 5g/l. Bacterial cultures were washed as above, and the pellet was mixed with ASM at a 1:1 ratio v/v before applying to the fused quartz slide.

Raman microspectroscopy experiments were conducted using a Renishaw InVia Raman microscope (Renishaw, UK), with a Leica DM 2500-M bright field microscope and an automated 100nm encoded XYZ stage. Samples were excited using 532nm and 785nm lasers directed through a Nikon 100x air objective (NA = 0.85), with collection after a Rayleigh edge filter appropriate to each excitation wavelength, and a diffraction grating (2400l/mm) that dispersed the Raman scattered light onto a Peltier cooled CCD (1024pixels x 256pixels). Calibration of the Raman shift was carried out using an internal silicon wafer. Spectra were acquired over three accumulations of 20 seconds.

All spectra were cleared of cosmic rays prior to analysis using Renishaw Wire 3.1 software, and then imported into iRootLab version 0.17.8.22-d for Matlab for further processing. Spectra were truncated to the 600-1600cm⁻¹ spectral region and then wavelet denoised to smooth them. To retain fluorescence (background) information, spectra were not background subtracted, but were normalised to their maximum intensity. PCA was applied to all datasets to reduce the dimensionality of the dataset. Either the raw data (for SVM), or the first ten PCs arising from PCA (for PCA-SVM) were fed into a SVM for classification, and ten-fold cross validation was used to validate the classifiers. For this study, iRootLab's in-built PCA, SVM, and k-fold cross-validation functionality were applied to the processed spectra to classify the bacteria by strain.

Raman spectroscopy has been shown to be able to differentiate between bacteria at the species and genus level [3, 1]. Using a single excitation wavelength, species often exhibit a large degree of spectral similarity, with many common features arising from shared biomolecules, such as DNA and amino acids.

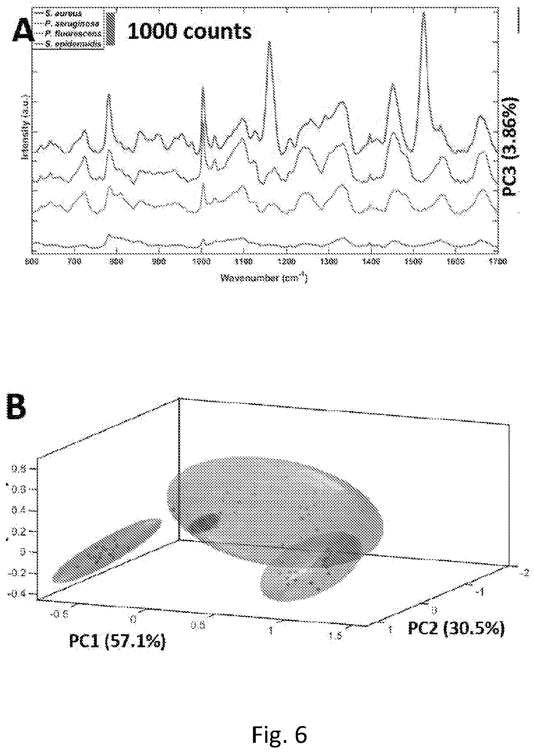

This can be seen in Fig. 6A, which shows (offset) the class mean spontaneous Raman spectra at 785nm excitation of four bacterial species: Staphylococcus aureus, Staphylococcus epidermidis, Pseudomonas aeruginosa, and Pseudomonas fluorescens. It can be seen that many bands are common to the spectra. Despite this similarity, it is possible to discriminate between spectra via the use of chemometric methods, such as unsupervised PCA.

Fig. 6B is a plot of the first three principal components, showing the clustering of bacterial spectra along species lines. Coloured envelopes represent the 95% confidence envelope for the corresponding species. This plot visually demonstrates the clustering of measured spectra in 3D PCA space based on genus and species. Clear grouping based on species is visible.

Despite some grouping visible with only a single excitation mode, the present method can be used to increase the spectral information content to improve identification and classification abilities. The present method utilises the differential enhancement of different peaks with different excitation modes. Differences in the peak ratios can be seen when the excitation wavelength is changed. This result is explained by the wavelength dependence of Raman cross-sections of vibrational modes.

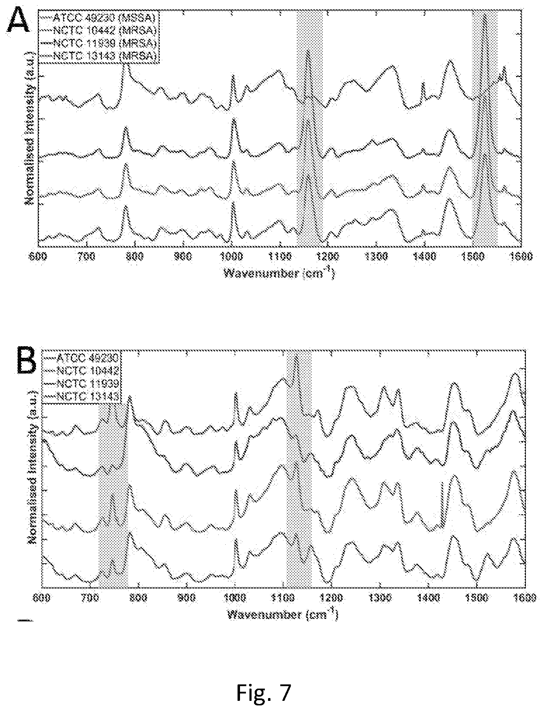

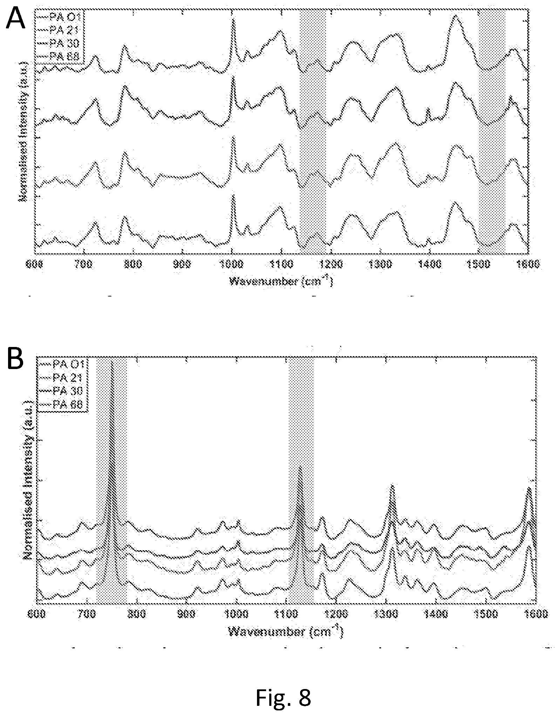

The differentially excited strong peaks are highlighted in Figs. 7 and 8. Fig. 7 shows normalised and offset spontaneous Raman spectra of S. aureus strains at excitation modes with wavelengths of (A) 785nm, and (B) 532nm. Fig. 8 shows normalised and offset spontaneous Raman spectra of P. aeruginosa strains at excitation modes with wavelengths of (A) 785nm, and (B) 532nm.

Grey highlights in Figs. 7 and 8 show regions of significant peak enhancement for each spectrum, illustrating the change in the magnitude of peaks. The 785nm excitation mode yields very strong peaks in SA that were absent in the spectra of PA. The 532nm excitation mode yields a different set of peaks were of very high intensity in PA which were significantly weaker in the four SA strains. Significant differences can also be seen between the excitation modes for the same bacterial species.

The differentially excited peaks, whether due to resonant/pre-resonant or non-resonant excitation, occur in different spectral regions and have different molecular origins. The strongly-enhanced vibrational modes around 750cm⁻¹ and 1120cm⁻¹ are attributed to ring breathing and half ring modes of porphyrins, which is consistent with the findings of Deng [16], and other groups that have probed porphyrin ring vibrations in Pseudomonas bacteria [17, 18].

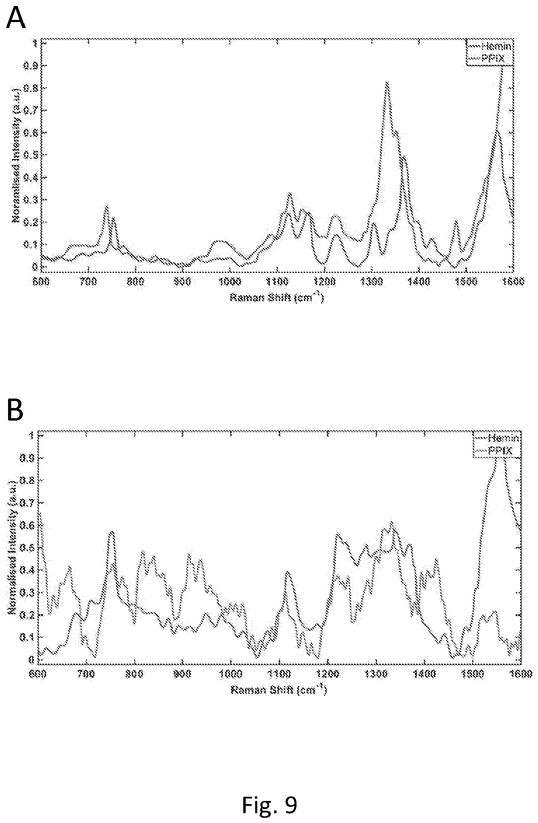

These are also consistent with the measured spectra of two different porphyrins in Fig. 9. Fig. 9A shows the Raman spectra of two porphyrin molecules: hemin and protoporphyrin IX, taken using 532nm wavelength of excitation (illumination) light from the illumination source 10. Fig. 9B shows the Raman spectra of hemin and protoporphyrin IX, taken using 785nm wavelength of excitation (illumination) light from the illumination source 10. The spectra clearly have peaks around 730-750cm⁻¹ and 1120cm⁻¹, confirming that the assignment to ring breathing and half-ring modes of the backbone, respectively, is consistent with resonance Raman studies on porphyrins [19, 20].

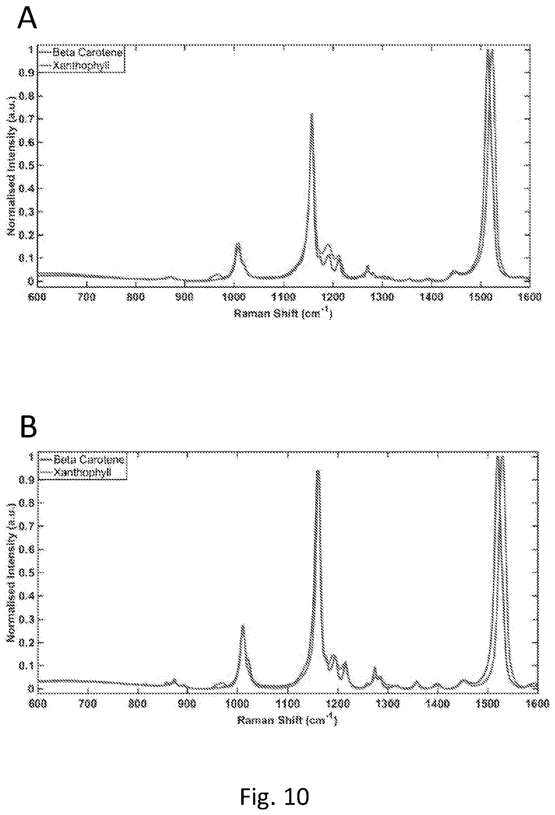

Similarly, the molecular origins of the enhanced bands at 1150cm⁻¹ and 1520cm⁻¹ in SA are attributed to C-C and C=C vibration modes present in carotenoids. This is also consistent with experimental spectra of typical carotenoids, shown in Fig. 10. Fig. 10A shows the Raman spectra of two carotenoid molecules: beta-carotene, and xanthophyll, taken using 532nm wavelength of excitation (illumination) light from the illumination source 10. Fig. 10B shows the Raman spectra of beta-carotene, and xanthophyll, taken using 785nm wavelength of excitation (illumination) light from the illumination source 10.

The spectra show characteristic peaks at ~1160cm⁻¹ and 1520cm⁻¹, arising from vibrations of the conjugated backbone of these molecules. The carotenoid peaks are assigned specifically to the presence of the pigment, staphyloxanthin, which is responsible for the yellow colour of SA strains. Further support for this assignment comes from the absence of the peaks in the spectra associated with strain NCTC 13143 (shown in Fig. 8A), which is derived from the drug resistant epidemic strain, EMRSA-16. This strain is white in appearance, and is known not to express staphyloxanthin [21]. These spectra are further consistent with published spectra, such as Naumann and Haung, who have observed these bands in biological samples [22, 23].

Of interest in these spectra is the observation of carotenoid pre-resonance at 785nm, and its absence at 532nm. It is common in studies of carotenoid species to use excitations in the range of 488nm, as these are known to provide strong enhancement of these molecules. However, the broad absorbance of many carotenoids extends up to around 500nm wavelengths [24-26], and should therefore be pre-resonant with 532nm excitation [24]. The use of 785nm excitation for this purpose is uncommon, but large Raman peaks associated with carotenoids in SA and a narrow subset of strains of the related S. epidermidis have been detected at 785nm [27].

To quantify the improvement in distinction ability using the combination of resonant/pre-resonant and non-resonant excitation, classification of spectra of different bacterial strains was carried out. A successful classification was defined as when a spectrum was within the 95% confidence interval for a strain. Any spectra that appeared outside of the confidence or within multiple confidence intervals of the same or different strains were regarded as unsuccessful.

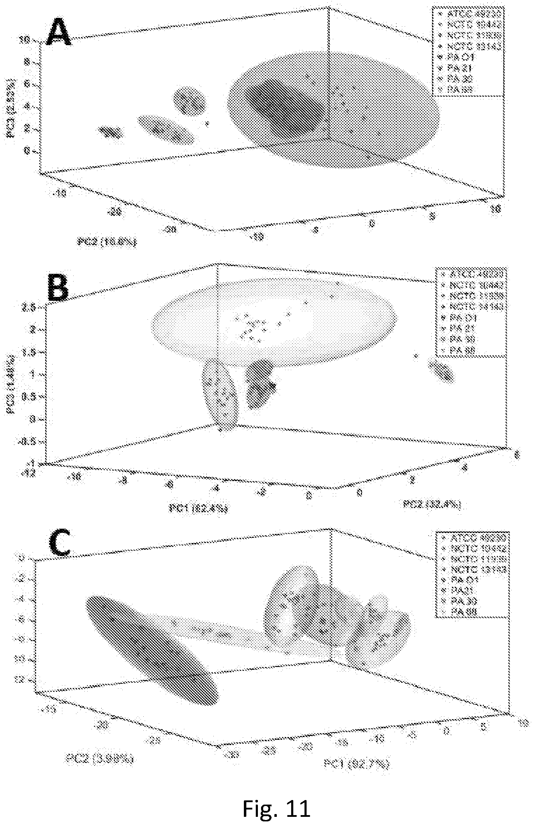

Fig. 11A shows a projection of the first three principal components for the measured spectra of 4 strains of S. aureus and 4 strains of P. aeruginosa taken with 785nm excitation mode alone. Clear separation of the four strains of SA is achieved, but poor separation of PA, leading to a poor classification accuracy of 60% across the 8 strains tested. Fig. 11B shows a projection of the first three principal components of the measured spectra of 4 strains of S. aureus and 4 strains of P. aeruginosa taken with 532nm excitation mode alone. Better separation of PA strains is achieved than of SA, but the classification accuracy is still relatively low, at 88.75%.

Using the two measured spectra from both excitation modes together according to the present method improves the classification accuracy. Both sets of normalised spectra were concatenated and PCA was performed. The projection of the first three principal components are shown in Fig. 11C. The classification accuracies improved to 93.75%. Table 2 shows the classification accuracies for the two excitation modes alone, and the two excitation modes together according to the present method. Additionally, accuracy of classification was higher when spectra were classified without polynomial background subtraction, indicating that the fluorescent background present in the Raman spectra encodes information that is useful in improving classification accuracy.

**Table 2**

| **Wavelength (nm)** | **Classification accuracy (%)** |
|---|---|
| 532 | 88.75 |
| 785 | 60 |
| 532+785 | 93.75 |

For diagnostic applications, an accuracy of 100% is highly desirable. To improve classification accuracy even further beyond the 93.75% already achieved, a more sophisticated classifier can be used. A support vector machine (SVM) was used, and PCA-SVM used for comparison. For PCA-SVM, ten principal components were established from the eight strains, for individual excitation modes having different wavelengths and for using the excitation modes together. These were fed into the SVM for classification. For SVM, 10-fold cross validation was used.

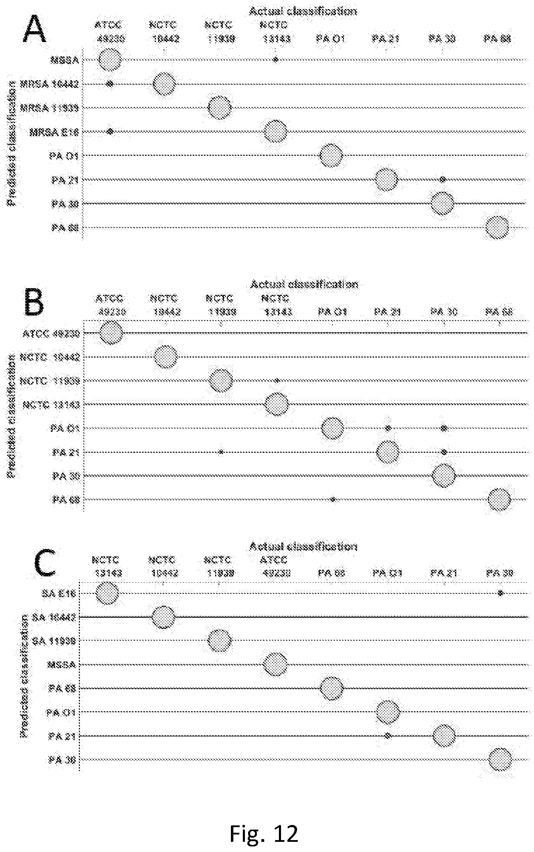

The classification accuracies for SVM of pure pellets of bacterial strains can be seen in Fig. 12. In each panel, the areas of the circles are proportional to the number of samples classified as that strain, with red circles representing misclassifications, and green circles showing correct classifications. Fig. 12A shows results for the 785nm excitation mode alone, Fig. 12B shows results for the 532nm excitation mode alone, and Fig. 12C shows results using both excitation modes together. The results show improved classification accuracy for using both excitation modes together, particularly in the case of P. aeruginosa strains.

Results for PCA-SVM offered lower accuracy than for SVM. This loss of accuracy relative to pure SVM is attributed to benefits of extra data points being available to SVM to aid in classification. In PCA-SVM, the data set only comprised ten principal components, whereas the number for the raw SVM is closer to 2500 observations at different wavenumbers, offering the trainer far more to learn from.

Classification accuracy was higher using SVM than PCA in all cases, with 785 nm alone achieving drastic improvements (97.52%), and smaller improvements for 532 nm alone (97.38%). Using the two measured spectra together offered some improvement over single excitation wavelength, achieving an accuracy of 98.64%.

The overall accuracy is already relatively high for the individual excitation modes used alone. However, using the excitation modes together does improve accuracy, and it is notable that there are fewer misclassifications with the combined excitation analysis. There were minimal inter-species classification errors, preserving the clinically-relevant distinction between the Gram-positive (SA) and Gram-negative (PA) species. Intra-species accuracies were also high, which is particularly important for pathogens such as SA. Only one of the four SA strains (ATCC 49230) shows methicillin sensitivity, and is reliably classified as distinct from its methicillin-resistant counterparts. This promising result indicates that the method can potentially identify drug resistance phenotypes in bacterial samples. In addition, there is greater genetic diversity across methicillin-sensitive S. aureus isolates than there is across MRSA lineages [28, 29], which further supports identification of antibiotic resistance by the described methodology.

Samples from patients, such as sputum, are not a sterile samples of pure bacteria. Therefore, further testing was carried out to validate the SVM embodiment of the present method in a more complex medium. Measured spectra were acquired with the bacterial strains mixed into artificial sputum medium (ASM), which was originally formulated to replicate the biochemical composition of CF sputum [30]. The choice of this medium was further informed by the use of this formulation in a variety of microbiological studies pertaining to PA strains [31, 32]. The samples were analysed with a 1:1 ratio of concentrated bacteria to ASM, which, while high, does not result in an unprecedented bacterial quantity. P. aeruginosa is commonly enumerated in CF sputum at concentrations of 10⁸-10¹⁰ CFU/ml, with Stressmann et al finding a maximum of 1.8*10¹¹ CFU/ml, in addition to a total bacterial density mean of approximately 10¹⁰ CFU/ml [34, 35].

The mixtures were prepared for analysis using a blood smear method, as already discussed in respect of Fig. 5, which is a common sample preparation method used in clinical laboratories. A reduction in the level of enhancement for the pre-resonant vibrational modes was observed in these bacteria-sputum samples, along with broad spectral features around 1300 cm⁻¹. These background features correspond to vibrational modes observed in the spectrum of pure ASM.

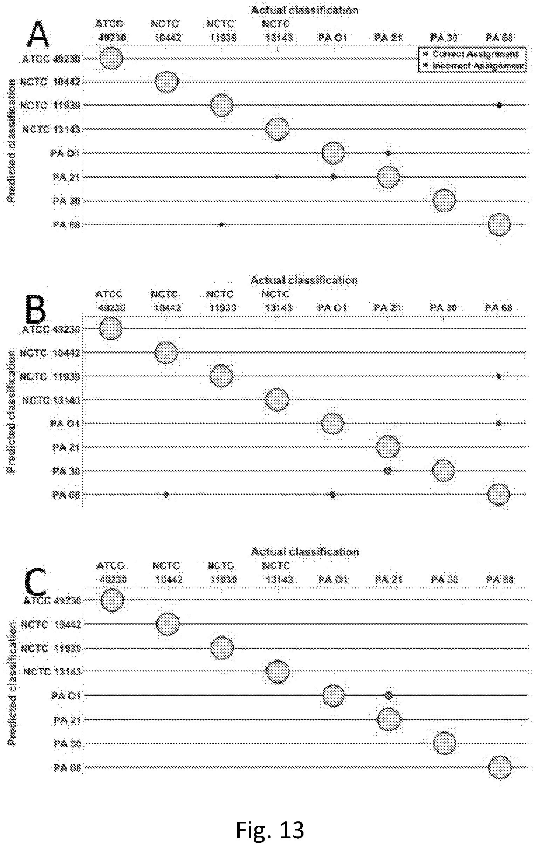

As with the pure bacterial pellets, SVM was performed using both the single excitation mode data and using both excitation modes together. The results are presented in Fig. 13. Fig. 13 shows the classification accuracies using SVM analysis of bacterial strains in ASM. Fig 13A shows results for the 785nm excitation mode alone, Fig. 13B shows results for the 532nm excitation mode alone, and Fig. 13C shows the results using both the measured spectra from both excitation modes (785 & 532nm) together. The size of green balls correspond to the number of correct identifications for that species. Red balls indicate incorrect classifications

High classification accuracies were achieved even in the presence of the interfering ASM. Classification accuracy of 94.38% was achieved for the 532 nm excitation mode alone, and 98.33% for the 785nm excitation mode alone. However, using both excitation modes together achieved a classification accuracy of 99.75%. It is also important to note that in the ASM experiment, the results using both excitation modes together showed no inter-species classification errors, and 100% accuracy for drug-sensitive and drug-resistant SA. This showed that the presence of the interfering media was no impediment to the discrimination of the drug-resistant and drug-sensitive strains of this pathogen. The results compare well to recent work by Ho et al, where deep learning was used for the identification of pathogenic bacteria based on their Raman spectra [33].

The present method relies only on the vibration of molecular bonds within the sample, which are consistent within the strains. The present method has considerable advantages in simplicity over MS-based methods, which require a dedicated expert and lab-based working environment. Given the commercial availability of a range of miniaturised and handheld Raman spectrometers, the present method is feasible for deployment on wards by existing clinical personnel.

At present, the priority of clinicians is most often the identification of the species of the pathogen rather than the strain. With the rising prevalence of drug resistant bacteria this strain-level differentiation ability is becoming increasingly clinically relevant. The high levels of accuracy in the experiments above in differentiating sensitive and resistant SA strains suggests that the method will be applicable to determining strain and serotype-linked anti-microbial resistance profiles of other pathogens. This method could help improve clinical outcomes, and enable the efficient targeting of treatments, by allowing for the monitoring of developing drug resistance in ongoing treatment, as well as aiding current efforts in anti-microbial stewardship. While experiments above demonstrate the method within the context of a CF model, the method is also applicable to a variety of other clinical scenarios and applications across sectors such as in food safety, where effective testing depends on rapidity and specificity.

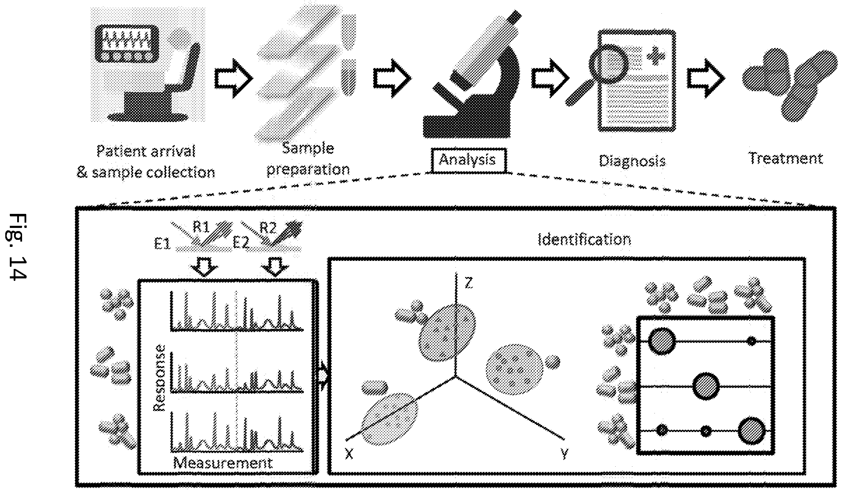

The present method can provide a rapid and reagentless diagnostic tool for clinicians, utilising simple and routine sample preparation that could be carried out on a ward. The envisaged workflow of such a device is shown in Fig. 14.

A patient arrives with a complaint and a biofluid sample is taken. The sample is prepared by a method analogous to a common blood smear, and placed into the apparatus for Raman analysis and subsequent classification. This classification then informs the clinician's final diagnosis and treatment options. The inset shows a representation of the Raman analysis. Measured spectra are obtained at multiple excitation modes that differ in wavelength and/or polarisation, and concatenated into a spectral matrix. This spectral-matrix is then fed into a trained PCA or LDA or SVM (or equivalent classification technique) model of known spectra for classification.

Applications exist across a number of sectors. The experiments above demonstrate the use to classify bacterial pathogens at the strain level in an artificial sputum growth media, including probing drug resistances (Methicillin-Resistant *S*. *aureus* vs sensitive strains). It could also be applied to the analysis of viral material and other biomolecules without patient samples, too, benefitting medical diagnoses as well as biomedical research.

The ability to probe different resonances and non-resonances may also be useful for the localisation of different bacterial species within co-species biofilms without the introduction of exogenous labels, such as fluorophores, which is of interest to microbiologists who explore the biofilm phenotype. Respiratory and urinary infections are also of particular relevance and interest as the analysis can be based on accessible biofuluids.

The method could also be applied to neurodiagnostics for neurodegenerative disorders, such as Alzheimer's disease. The present method could detect precursors of Alzheimer's and other neurodegenerative diseases before the onset of physiological symptoms. This, combined with informed interpretation of signals and with input from clinicians, will enable a new method for all-optical and non-invasive or minimally-invasive diagnosis of neurodegenerative diseases. This could be based on analysis of sasliva, blood, or other biofluids such as cerebrospinal fluid (CSF).

The method could also be applied to screening patients for drug trials, especially in the area of neurodegenerative diseases where there is a significant heterogeneity in patients and their disease sub-types. This is a significant reason for failure of many clinical trials of potential drugs. The present method can be applied to screen patients using biofluids such as blood, serum, cerebrospinal fluid etc., as well as to screen drugs in pre-clinical models. The method could also be used for monitoring drug action in pre-clinical models or in patients before or during drug trials and beyond for treatment monitoring.

Applications also exist where it is desirable to accurately identify biomaterials in complex matrices. For example, applications exist in water treatment, food and pharmaceutical quality control, and screening for contaminants that represent a public health concern, or which may lead to premature spoilage of products. Biofouling of pipelines is also an issue for the petrochemical industry, and the present method would also facilitate simpler monitoring of fouling at the point of analysis, rather than requiring longer lab work-ups.

Applications will also be found in military, homeland security, and forensic applications, in the identification of a variety of unknowns without direct contact with the sample. Analysis of materials such as explosives and accelerants would benefit from noncontact identification methods.

### References

[1] Kloß, S., P. Rösch, W. Pfister, M. Kiehntopf and J. Popp (2015). "Toward Culture-Free Raman Spectroscopic Identification of Pathogens in Ascitic Fluid." Analytical Chemistry 87(2): 937-943.
[2] Kloß, S., B. Lorenz, S. Dees, I. Labugger, P. Rösch and J. Popp (2015). "Destruction-free procedure for the isolation of bacteria from sputum samples for Raman spectroscopic analysis." Analytical and Bioanalytical Chemistry 407(27): 8333-8341.
[3] Ghebremedhin, M., R. Heitkamp, S. Yesupriya, B. Clay and N. J. Crane (2017). "Accurate and Rapid Differentiation of <span class="named-content genus-species" id="named-content-1">Acinetobacter baumannii</span> Strains by Raman Spectroscopy: a Comparative Study." Journal of Clinical Microbiology 55(8): 2480-2490.
[4] Li, J., C. Wang, H. Kang, L. Shao, L. Hu, R. Xiao, S. Wang and B. Gu (2018). "Label-free identification carbapenem-resistant Escherichia coli based on surface-enhanced resonance Raman scattering." RSC Advances 8(9): 4761-4765.
[5] Allen, D., J. Kelly, D. Gilpin, S. Bell and M. Tunney (2019). "Detection and characterisation of bacteria causing lung infection in people with Cystic Fibrosis (CF) by surface-enhanced Raman spectroscopy (SERS)." Access Microbiology 1(1A).
[6] Polisetti, S., N. F. Baig, N. Morales-Soto, J. D. Shrout and P. W. Bohn (2017). "Spatial Mapping of Pyocyanin in Pseudomonas Aeruginosa Bacterial Communities Using Surface Enhanced Raman Scattering." Applied Spectroscopy 71(2): 215-223.
[7] R. M. Jarvis and R. Goodacre, Analytical Chemistry, 2004, 76, 40-47.
[8] R. M. Jarvis and R. Goodacre, Chemical Society Reviews, 2008, 37, 931-936.
[9] W. R. Premasiri, J. C. Lee, A. Sauer-Budge, R. Théberge, C. E. Costello and L. D. Ziegler, Analytical and Bioanalytical Chemistry, 2016, 408, 4631-4647.
[10] L. F. Tadesse, F. Safir, C.-S. Ho, X. Hasbach, B. Khuri-Yakub, S. S. Jeffrey, A. A. E. Saleh and J. Dionne, The Journal of Chemical Physics, 2020, 152, 240902.
[11] B. Yellampelle, M. Sluch, S. Asher and B. Lemoff, Multiple-excitation-wavelength resonance-Raman explosives detection, SPIE, 2011.
[12] Asher, S. A. (1993). "UV resonance Raman spectroscopy for analytical, physical, and biophysical chemistry. Part 2." Analytical chemistry 65(4): 201A-210A.
[13] Cotton, T. M., R. Timkovich and M. S. Cork (1981). "Resonance Raman and surface-enhanced resonance Raman studies of cytochrome cd 1." FEBS Letters 133(1): 39-44.
[14] Manoharan, R., E. Ghiamati, R. A. Dalterio, K. A. Britton, W. H. Nelson and J. F. Sperry (1990). "UV resonance Raman spectra of bacteria, bacterial spores, protoplasts and calcium dipicolinate." Journal of Microbiological Methods 11(1): 1-15.
[15] Rusciano, G. C., P.; Pesce, G.; Abete, P.; Carnovale, V.; Sasso, A. (2013). "Raman spectroscopy as a new tool for early detection of bacteria in patients with cystic fibrosis." Laser Physics Letters 10(7).
[16] J. L. Deng, Q. Wei, M. H. Zhang, Y. Z. Wang and Y. Q. Li, Journal of Raman Spectroscopy, 2005, 36, 257-261.
[17] S. Berezhna, H. Wohlrab and P. M. Champion, Biochemistry, 2003, 42, 6149-6158.
[18] M. Rönnberg, K. Osterlund and N. Ellfolk, Biochimica et Biophysica Acta (BBA) - Protein Structure, 1980, 626, 23-30.
[19] A. Gorski, A. Starukhin, S. Stavrov, S. Gawinkowski and J. Waluk, Spectrochimica Acta Part A: Molecular and Biomolecular Spectroscopy, 2017, 173, 350-355.
[20] T. G. Spiro and T. C. Strekas, Proceedings of the National Academy of Sciences, 1972, 69, 2622.
[21] P. R. McAdam, K. E. Templeton, G. F. Edwards, M. T. G. Holden, E. J. Feil, D. M. Aanensen, H. J. A. Bargawi, B. G. Spratt, S. D. Bentley, J. Parkhill, M. C. Enright, A. Holmes, E. K. Girvan, P. A. Godfrey, M. Feldgarden, A. M. Kearns, A. Rambaut, D. A. Robinson and J. R. Fitzgerald, Proceedings of the National Academy of Sciences, 2012, 109, 9107.
[22] D. Naumann, Infrared and NIR Raman spectroscopy in medical microbiology, SPIE, 1998.
[23] Z. Huang, A. McWilliams, H. Lui, D. I. McLean, S. Lam and H. Zeng, International Journal of Cancer, 2003, 107, 1047-1052.
[24] V. E. Igor, S. Mohsen, R. E. Maia and G. Werner, BIOMEDO, 2005, 10, 1-18.
[25] P. S. Bernstein, D.-Y. Zhao, S. W. Wintch, I. V. Ermakov, R. W. McClane and W. Gellermann, Ophthalmology, 2002, 109, 1780-1787.
[26] J. C. Merlin, Pure and Applied Chemistry, 1985, 57, 785-792.
[27] K. Rebrošová, M. Šiler, O. Samek, F. Růžička, S. Bernatová, J. Ježek, P. Zemánek and V. Holá, Future Microbiology, 2017, 12, 881-890.
[28] J. A. Lindsay, International Journal of Medical Microbiology, 2010, 300, 98-103.
[29] D. Patel, M. J. Ellington, R. Hope, R. Reynolds, C. Arnold and M. Desai, Journal of Hospital Infection, 2015, 91, 136-145.
[30] D. D. Sriramulu, H. Lünsdorf, J. S. Lam and U. Römling, Journal of Medical Microbiology, 2005, 54, 667-676.
[31] V. Behrends, B. Geier, H. D. Williams and J. G. Bundy, Applied and Environmental Microbiology, 2013, 79, 2467.
[32] S. Kirchner, J. L. Fothergill, E. A. Wright, C. E. James, E. Mowat and C. Winstanley, JoVE, 2012, DOI: doi:10.3791/3857, e3857.
[33] C.-S. Ho, N. Jean, C. A. Hogan, L. Blackmon, S. S. Jeffrey, M. Holodniy, N. Banaei, A. A. E. Saleh, S. Ermon and J. Dionne, Nature Communications, 2019, 10, 4927.
[34] K. L. Palmer, L. M. Mashburn, P. K. Singh and M. Whiteley, J Bacteriol, 2005, 187, 5267-5277.
[35] F. A. Stressmann, G. B. Rogers, P. Marsh, A. K. Lilley, T. W. V. Daniels, M. P. Carroll, L. R. Hoffman, G. Jones, C. E. Allen, N. Patel, B. Forbes, A. Tuck and K. D. Bruce, Journal of Cystic Fibrosis, 2011, 10, 357-365.
[36] J. Grun, C. K. Manka, S. Nikitin, D. Zabetakis, G. Comanescu, D. Gillis, and J. Bowles, Analytical Chemistry, 2007, vol. 79, no. 14, pages 5489-5493, DOI: doi: 10.1021/ac070681h.

## Claims

1. A method of identifying one or more substances in a sample (3) comprising:
illuminating the sample (3) with light of each of a plurality of different excitation modes;
measuring an intensity of light from the sample (3) at a plurality of wavelengths to obtain a measured Raman spectrum for each of the excitation modes; and
identifying one or more substances in the sample (3) using the measured spectra together, wherein:
the excitation modes differ in wavelength;
and **characterised in that**
the wavelengths of the excitation modes are such that the measured spectra comprise two or more of a non-resonant Raman spectrum, a pre-resonant Raman spectrum, and a resonant Raman spectrum; and
the identifying of the one or more substances uses contributions to the measured spectra from a plurality of photophysical processes in the sample (3) including Raman scattering of light and fluorescence.

2. The method of claim 1, wherein identifying the one or more substances comprises one or more of:
a) using a multivariate analysis, for example principal component analysis or linear discriminant analysis, optionally wherein identifying the one or more substances comprises combining the measured spectra using the multivariate analysis to obtain a multi-dimensional signature of the sample, further optionally wherein identifying the one or more substances further comprises comparing the multi-dimensional signature to one or more reference signatures;
b) using a machine-learning algorithm, for example a support vector machine or a neural network; and
c) classifying the one or more substances.

3. The method of any preceding claim, wherein identifying the one or more substances does not comprise:
a) processing to reduce the contribution to the measured spectra of any photophysical processes in the sample (3); and/or
b) processing to reduce the contribution to the measured spectra of the fluorescence in the sample (3).

4. The method of any preceding claim, wherein one or both of:
a) a polarisation of at least one of the excitation modes comprises linear polarisation, circular polarisation, or elliptical polarisation; and
b) two or more of the excitation modes differ from one another in polarisation.

5. The method of any preceding claim, wherein one or more of:
a) the wavelengths of the excitation modes comprise one or more visible light wavelengths, optionally wherein the one or more visible light wavelengths comprise a wavelength in the range 400-700nm, further optionally wherein the wavelengths of the excitation modes comprise one or more of 405nm, 532nm, and 633nm;
b) the wavelengths of the excitation modes comprise one or more infra-red light wavelengths, optionally wherein the one or more infra-red light wavelengths comprise a wavelength in the range 700-3000nm, further optionally wherein the wavelengths of the excitation modes comprise one or more of 785nm and 1064nm; and
c) two or more of the excitation modes differ from one another in wavelength by at least 20nm.

6. The method of any preceding claim, wherein either:
a) illuminating the sample (3) with light of each of a plurality of different excitation modes comprises illuminating the sample (3) simultaneously with light of each of the excitation modes; or
b) illuminating the sample (3) with light of each of a plurality of different excitation modes comprises illuminating the sample (3) sequentially with light of each of the excitation modes.

7. The method of any preceding claim, wherein the one or more substances comprise microorganisms, for example bacteria or archaea, and identifying the one or more substances comprises classifying the microorganisms,
optionally wherein classifying the microorganisms comprises identifying a category of the microorganisms, wherein the category comprises one or more of a taxonomic group of the microorganisms and a phenotype of the microorganisms,
further optionally wherein one or both of: a) the taxonomic group comprises a sub-species, strain, species, genus, family, order, or class; and b) the phenotype comprises a type of anti-microbial resistance or anti-biotic susceptibility,
further optionally wherein the sample (3) comprises microorganisms of two or more categories, and identifying the one or more substances comprises determining a quantity and/or relative proportion of microorganisms of each of the two or more categories.

8. The method of any preceding claim, wherein the one or more substances comprise any type of amyloid fibrils, amyloid plaques or its precursors, tau and phospho-tau, huntingtin, other markers of proteinopathies, neurofilament light chain, or extracellular matrix components, optionally collagen and/or elastin.

9. The method of any preceding claim, wherein the sample (3) is a biological sample, optionally wherein the sample (3) is obtained from an organism, and the method further comprises determining a type, a likelihood, a severity, and/or a stage of a disease or condition for the organism on the basis of the identification of the one or more substances, further optionally wherein the organism is a human.

10. The method of any of claims 1 to 8, wherein the sample (3) is obtained from a non-biological source,
optionally wherein the method further comprises determining a likelihood or severity of contamination of the non-biological source on the basis of the identification of the one or more substances,
further optionally wherein the contamination is a biological contamination.

11. An apparatus (1) for identifying one or more substances in a sample (3) comprising:
an illumination source (10) configured to illuminate the sample (3) with light of each of a plurality of different excitation modes;
a detector (20) configured to measure an intensity of light from the sample (3) at a plurality of wavelengths to obtain a measured Raman spectrum for each of the excitation modes; and
a processing unit (30) configured to identify one or more substances in the sample (3) using the measured spectra together, wherein:
the excitation modes differ in wavelength;
and **characterised in that**
the wavelengths of the excitation modes are such that the measured spectra comprise two or more of a non-resonant Raman spectrum, a pre-resonant Raman spectrum, and a resonant Raman spectrum; and
the processing unit (30) is configured to identify the one or more substances using contributions to the measured spectra from a plurality of photophysical processes in the sample including Raman scattering of light and fluorescence.

12. The apparatus (1) of claim 11, wherein the illumination source (10) comprises one or both of:
a) a wavelength-tuneable illumination source; and
b) a plurality of sub-sources (12), each sub-source (12) configured to emit light at a different wavelength from the other sub-sources (12)

13. The apparatus (1) of any of claims 11 or 12, wherein one or both of
a) the illumination source (10) is configured to emit polarised light, optionally wherein i) the polarisation of the light emitted by the illumination source (10) is tuneable, and/or ii) the illumination source (10) comprises a plurality of sub-sources (12), each sub-source (12) configured to emit light having a different polarisation from the other sub-sources (12); and
b) the bandwidth of light emitted by the illumination source (10) is sufficiently narrow to resolve a Raman linewidth of 50 cm⁻¹ or less.

14. The apparatus (1) of any of claims 11 to 13, wherein either:
a) the detector (20) is configured to detect light reflected and/or backscattered from the sample (3); or
b) the detector (20) is configured to detect light transmitted through and/or scattered by the sample (3).

15. The apparatus (1) of any of claims 11 to 14, wherein the apparatus (1) further comprises one or more of:
a) a filtering element (50) configured to remove light at the wavelength of the excitation mode from the light from the sample (3), optionally wherein the filtering element (50) comprises a shortpass optical filter, longpass optical filter, notch optical filter, bandpass optical filter, or electro-optical modulator;
b) a detection polariser (52) configured to select light having a predetermined polarisation from the light from the sample (3); and
c) a resolving element (54) configured to spectrally resolve the light from the sample (3), optionally wherein the resolving element (54) comprises a spectrograph, a grating, a prism, or an interferometer.

## Patentansprüche

1. Verfahren zum Identifizieren einer oder mehrerer Substanzen in einer Probe (3), das umfasst:
Beleuchten der Probe (3) mit Licht jedes einer Vielzahl verschiedener Anregungsmodi;
Messen der Lichtintensität der Probe (3) bei einer Vielzahl von Wellenlängen, um für jeden der Anregungsmodi ein gemessenes Raman-Spektrum zu erhalten; und
Identifizieren einer oder mehrerer Substanzen in der Probe (3) unter gemeinsamer Verwendung der gemessenen Spektren, wobei:
sich die Anregungsmodi in der Wellenlänge unterscheiden;
und **dadurch gekennzeichnet, dass**
die Wellenlängen der Anregungsmodi so gewählt sind, dass die gemessenen Spektren zwei oder mehr der Folgenden umfassen: ein nicht-resonantes Raman-Spektrum, ein prä-resonantes Raman-Spektrum und ein resonantes Raman-Spektrum; und
die Identifizierung der einen oder mehreren Substanzen Beiträge zu den gemessenen Spektren aus einer Vielzahl von photophysikalischen Prozessen in der Probe (3) enthält, einschließlich Raman-Streuung von Licht und Fluoreszenz.

2. Verfahren nach Anspruch 1, wobei die Identifizierung der einen oder mehreren Substanzen eines oder mehrere der Folgenden umfasst:
a) Verwenden einer multivariaten Analyse, beispielsweise einer Hauptkomponentenanalyse oder einer linearen Diskriminanzanalyse, optional wobei die Identifizierung der einen oder mehreren Substanzen das Kombinieren der gemessenen Spektren unter Verwendung der multivariaten Analyse umfasst, um eine mehrdimensionale Signatur der Probe zu erhalten, ferner optional wobei die Identifizierung der einen oder mehreren Substanzen ferner das Vergleichen der mehrdimensionalen Signatur mit einer oder mehreren Referenzsignaturen umfasst;
b) Verwenden eines Algorithmus des maschinellen Lernens, beispielsweise einer Support-Vektor-Maschine oder eines neuronalen Netzwerks; und
c) Klassifizieren der einen oder mehreren Substanzen.

3. Verfahren nach einem der vorhergehenden Ansprüche, wobei das Identifizieren der einen oder mehreren Substanzen nicht umfasst:
a) Verarbeiten zur Verringerung des Beitrags photophysikalischer Prozesse in der Probe (3) zu den gemessenen Spektren; und/oder
b) Verarbeiten zur Verringerung des Beitrags der Fluoreszenz in der Probe (3) zu den gemessenen Spektren.

4. Verfahren nach einem der vorhergehenden Ansprüche, wobei eines oder beide der Folgenden zutreffen:
a) eine Polarisation mindestens eines der Anregungsmodi umfasst lineare Polarisation, zirkulare Polarisation oder elliptische Polarisation; und
b) zwei oder mehr der Anregungsmodi unterscheiden sich in ihrer Polarisation voneinander.

5. Verfahren nach einem der vorhergehenden Ansprüche, wobei eines oder mehrere der Folgenden zutreffen:
a) die Wellenlängen der Anregungsmoden umfassen eine oder mehrere Wellenlängen des sichtbaren Lichts, optional wobei die eine oder mehrere Wellenlängen des sichtbaren Lichts eine Wellenlänge im Bereich von 400-700 nm umfassen, ferner optional wobei die Wellenlängen der Anregungsmoden eine oder mehrere der Wellenlängen 405 nm, 532 nm und 633 nm umfassen;
b) die Wellenlängen der Anregungsmoden umfassen eine oder mehrere Wellenlängen im Infrarotbereich, optional wobei die eine oder mehrere Wellenlängen im Infrarotbereich eine Wellenlänge im Bereich von 700 bis 3000 nm umfassen, ferner optional wobei die Wellenlängen der Anregungsmoden eine oder mehrere der Wellenlängen 785 nm und 1064 nm umfassen; und
c) sich zwei oder mehr der Anregungsmodi in der Wellenlänge um mindestens 20 nm voneinander unterscheiden.

6. Verfahren nach einem der vorhergehenden Ansprüche, wobei entweder:
a) das Bestrahlen der Probe (3) mit Licht jedes einer Vielzahl verschiedener Anregungsmodi das gleichzeitige Bestrahlen der Probe (3) mit Licht jedes der Anregungsmodi umfasst; oder
b) das Bestrahlen der Probe (3) mit Licht jedes einer Vielzahl verschiedener Anregungsmodi das sequenzielle Bestrahlen der Probe (3) mit Licht jedes der Anregungsmodi umfasst.

7. Verfahren nach einem der vorhergehenden Ansprüche, wobei die eine oder mehreren Substanzen Mikroorganismen, beispielsweise Bakterien oder Archaea, umfassen und das Identifizieren der einen oder mehreren Substanzen das Klassifizieren der Mikroorganismen umfasst,
wobei optional das Klassifizieren der Mikroorganismen das Identifizieren einer Kategorie der Mikroorganismen umfasst, wobei die Kategorie eines oder mehrere der Folgenden umfasst: eine taxonomische Gruppe der Mikroorganismen und einen Phänotyp der Mikroorganismen,
wobei ferner optional eines oder beide der Folgenden zutreffen: a) die taxonomische Gruppe umfasst eine Unterart, einen Stamm, eine Art, eine Gattung, eine Familie, eine Ordnung oder eine Klasse; und b) der Phänotyp umfasst eine Art von antimikrobieller Resistenz oder Antibiotikaempfindlichkeit,
wobei ferner optional die Probe (3) Mikroorganismen aus zwei oder mehr Kategorien umfasst, und das Identifizieren der einen oder mehreren Substanzen das Bestimmen einer Menge und/oder eines relativen Anteils an Mikroorganismen jeder der zwei oder mehr Kategorien umfasst.

8. Verfahren nach einem der vorhergehenden Ansprüche, wobei die eine oder mehreren Substanzen jede Art von Amyloidfibrillen, Amyloidplaques oder deren Vorläufer, Tau und Phospho-Tau, Huntingtin, andere Marker für Proteinopathien, die leichte Kette des Neurofilaments oder Komponenten der extrazellulären Matrix, optional Kollagen und/oder Elastin, umfassen.

9. Verfahren nach einem der vorhergehenden Ansprüche, wobei die Probe (3) eine biologische Probe ist,
wobei die Probe (3) optional von einem Organismus gewonnen wird, und das Verfahren ferner die Bestimmung einer Art, einer Wahrscheinlichkeit, eines Schweregrads und/oder eines Stadiums einer Erkrankung oder eines Zustands des Organismus basierend auf der Identifizierung der einen oder mehreren Substanzen umfasst,
wobei optional der Organismus ein Mensch ist.

10. Verfahren nach einem der Ansprüche 1 bis 8, wobei die Probe (3) aus einer nicht-biologischen Quelle gewonnen wird,
wobei das Verfahren optional ferner die Bestimmung einer Wahrscheinlichkeit oder eines Schweregrads einer Kontamination der nicht-biologischen Quelle basierend auf der Identifizierung der einen oder mehreren Substanzen umfasst,
wobei die Kontamination ferner optional eine biologische Kontamination ist.

11. Vorrichtung (1) zum Identifizieren einer oder mehrerer Substanzen in einer Probe (3), das umfasst:
eine Beleuchtungsquelle (10), die so konfiguriert ist, dass sie die Probe (3) mit Licht jedes einer Vielzahl verschiedener Anregungsmodi beleuchtet;
einen Detektor (20), der so konfiguriert ist, dass er die Lichtintensität der Probe (3) bei einer Vielzahl von Wellenlängen misst, um für jeden der Anregungsmodi ein gemessenes Raman-Spektrum zu erhalten; und
eine Verarbeitungseinheit (30), die so konfiguriert ist, dass sie eine oder mehrere Substanzen in der Probe (3) anhand der gemessenen Spektren gemeinsam identifiziert, wobei:
sich die Anregungsmodi in der Wellenlänge unterscheiden;
und **dadurch gekennzeichnet, dass**
die Wellenlängen der Anregungsmodi so gewählt sind, dass die gemessenen Spektren zwei oder mehr der Folgenden umfassen: ein nicht-resonantes Raman-Spektrum, ein prä-resonantes Raman-Spektrum und ein resonantes Raman-Spektrum; und
die Verarbeitungseinheit (30) so konfiguriert ist, dass sie die eine oder mehreren Substanzen unter Verwendung von Beiträgen zu den gemessenen Spektren aus einer Vielzahl von photophysikalischen Prozessen in der Probe, die Raman-Streuung von Licht und Fluoreszenz enthalten, identifizieren.

12. Vorrichtung (1) nach Anspruch 11, wobei die Beleuchtungsquelle (10) eines oder beides der Folgenden umfasst:
a) eine wellenlängenabstimmbare Beleuchtungsquelle; und
b) eine Vielzahl von Teilquellen (12), wobei jede Teilquelle (12) so konfiguriert ist, dass sie Licht bei einer anderen Wellenlänge als die anderen Teilquellen (12) emittiert.

13. Vorrichtung (1) nach einem der Ansprüche 11 oder 12, wobei eines oder beide der Folgenden zutreffen
a) die Beleuchtungsquelle (10) ist so konfiguriert, dass sie polarisiertes Licht aussendet, optional wobei i) die Polarisation des von der Beleuchtungsquelle (10) emittierten Lichts abstimmbar ist, und/oder ii) die Beleuchtungsquelle (10) eine Vielzahl von Teilquellen (12) umfasst, wobei jede Teilquelle (12) so konfiguriert ist, dass sie Licht mit einer anderen Polarisation als die anderen Teilquellen (12) emittiert; und
b) die Bandbreite des von der Beleuchtungsquelle (10) emittierten Lichts ausreichend schmal ist, um eine Raman-Linienbreite von 50 cm⁻¹ oder weniger aufzulösen.

14. Vorrichtung (1) nach einem der Ansprüche 11 bis 13, wobei entweder
a) der Detektor (20) so konfiguriert ist, dass er von der Probe (3) reflektiertes und/oder rückgestreutes Licht erfasst; oder
b) der Detektor (20) so konfiguriert ist, dass er Licht erfasst, das durch die Probe (3) hindurchgelassen und/oder von dieser gestreut wird.

15. Vorrichtung (1) nach einem der Ansprüche 11 bis 14, wobei die Vorrichtung (1) ferner eines oder mehrere der Folgenden umfasst:
a) ein Filterelement (50), das so konfiguriert ist, dass es Licht mit der Wellenlänge des Anregungsmodus aus dem Licht der Probe (3) entfernt, wobei das Filterelement (50) optional ein optisches Kurzpassfilter, ein optisches Langpassfilter, ein optisches Kerbfilter, ein optisches Bandpassfilter oder einen elektrooptischen Modulator umfasst;
b) einen Detektionspolarisator (52), der so konfiguriert ist, dass er Licht mit einer vorbestimmten Polarisation aus dem Licht der Probe (3) auswählt; und
c) ein Auflösungselement (54), das so konfiguriert ist, dass es das Licht der Probe (3) spektral auflöst,
wobei das Auflösungselement (54) optional einen Spektrographen, ein Gitter, ein Prisma oder ein Interferometer umfasst.

## Revendications

1. Procédé d'identification d'une ou plusieurs substances dans un échantillon (3), comprenant :
l'éclairage de l'échantillon (3) avec une lumière d'une pluralité de modes d'excitation différents,
la mesure de l'intensité lumineuse provenant de l'échantillon (3) à une pluralité de longueurs d'onde pour obtenir un spectre Raman mesuré pour chacun des modes d'excitation, et
l'identification d'une ou plusieurs substances dans l'échantillon (3) en utilisant conjointement lesdits spectres mesurés ; étant entendu que :
les modes d'excitation sont de longueurs d'onde différentes ;
et **caractérisé en ce que** :
les longueurs d'onde des modes d'excitation sont telles que les spectres mesurés comprennent au moins deux spectres parmi un spectre Raman non résonant, un spectre Raman pré-résonant et un spectre Raman résonant, et
l'identification de la ou des substances utilise les contributions aux spectres mesurés d'une pluralité de processus photophysiques de l'échantillon (3), dont la diffusion Raman de la lumière et la fluorescence.

2. Procédé selon la revendication 1, dans lequel l'identification de la ou des substances comprend une ou plusieurs des opérations suivantes :
a) l'utilisation d'une analyse multivariée, par exemple une analyse en composantes principales ou une analyse discriminante linéaire, l'identification de la ou des substances comprenant éventuellement la combinaison, à l'aide de l'analyse multivariée, des spectres mesurés pour obtenir une signature multidimensionnelle de l'échantillon, auquel cas l'identification de la ou des substances comprend éventuellement en outre la comparaison de la signature multidimensionnelle à une ou plusieurs signatures de référence,
b) l'utilisation d'un algorithme d'apprentissage automatique, par exemple une machine à vecteurs de support ou un réseau de neurones, et
c) le classement de la ou des substances.

3. Procédé selon l'une quelconque des revendications précédentes, dans lequel l'identification de la ou des substances ne comprend pas :
a) un traitement visant à réduire la contribution aux spectres mesurés de tout processus photophysique de l'échantillon (3), et/ou
b) un traitement visant à réduire la contribution aux spectres mesurés de la fluorescence de l'échantillon (3).

4. Procédé selon l'une quelconque des revendications précédentes, dans lequel :
a) la polarisation d'au moins l'un des modes d'excitation comprend une polarisation linéaire, une polarisation circulaire ou une polarisation elliptique, et/ou
b) au moins deux des modes d'excitation ont des polarisations différentes.

5. Procédé selon l'une quelconque des revendications précédentes, dans lequel :
a) les longueurs d'onde des modes d'excitation comprennent une ou plusieurs longueurs d'onde de lumière visible, la ou les longueurs d'onde de lumière visible comprenant éventuellement une longueur d'onde située dans la plage de 400 à 700 nm, lesdites longueurs d'onde des modes d'excitation étant en outre éventuellement de 405 nm, 532 nm et/ou 633 nm, et/ou
b) les longueurs d'onde des modes d'excitation comprennent une ou plusieurs longueurs d'onde de lumière infrarouge, la ou lesdites longueurs d'onde de lumière infrarouge comprenant éventuellement une longueur d'onde située dans la plage de 700 à 3000 nm, lesdites longueurs d'onde des modes d'excitation étant en outre éventuellement de 785 nm et/ou 1064 nm, et/ou
c) au moins deux des modes d'excitation ont des longueurs d'onde qui diffèrent d'au moins 20 nm.

6. Procédé selon l'une quelconque des revendications précédentes, dans lequel soit :
a) l'éclairage de l'échantillon (3) avec une lumière d'une pluralité de modes d'excitation différents comprend l'éclairage de l'échantillon (3) simultanément avec une lumière de chacun des modes d'excitation, soit
b) l'éclairage de l'échantillon (3) avec une lumière d'une pluralité de modes d'excitation différents comprend l'éclairage de l'échantillon (3) séquentiellement avec une lumière de chacun des modes d'excitation.

7. Procédé selon l'une quelconque des revendications précédentes, dans lequel la ou les substances comprennent des micro-organismes, par exemple des bactéries ou des archées, et l'identification de la ou des substances comprend le classement des micro-organismes,
ledit classement des micro-organismes comprenant éventuellement l'identification de la catégorie des micro-organismes, ladite catégorie comprenant le groupe taxonomique des micro-organismes et/ou le phénotype des micro-organismes,
auquel cas, en outre : a) le groupe taxonomique comprend éventuellement une sousespèce, une souche, une espèce, un genre, une famille, un ordre ou une classe ; et/ou b) le phénotype comprend éventuellement un type de résistance aux antimicrobiens ou de sensibilité aux antibiotiques,
ledit échantillon (3) comprenant en outre éventuellement des micro-organismes d'au moins deux catégories et l'identification de la ou des substances comprenant la détermination de la quantité et/ou de la proportion relative de micro-organismes de chacune des au moins deux catégories.

8. Procédé selon l'une quelconque des revendications précédentes, dans lequel la ou les substances comprennent tout type de fibrilles amyloïdes, plaques amyloïdes ou leurs précurseurs, la protéine tau et phospho-tau, la huntingtine, d'autres marqueurs de protéinopathies, la chaîne légère de neurofilament ou des composants de la matrice extracellulaire, éventuellement le collagène et/ou l'élastine.

9. Procédé selon l'une quelconque des revendications précédentes, dans lequel l'échantillon (3) est un échantillon biologique, ledit échantillon (3) étant éventuellement obtenu à partir d'un organisme, ledit procédé comprenant en outre la détermination du type, de la probabilité, de la gravité et/ou du stade d'une maladie ou d'une affection propre à l'organisme compte tenu de l'identification de la ou des substances, ledit organisme étant en outre éventuellement un être humain.

10. Procédé selon l'une quelconque des revendications 1 à 8, dans lequel l'échantillon (3) est obtenu à partir d'une source non biologique,
ledit procédé comprenant en outre éventuellement la détermination de la probabilité ou de la gravité d'une contamination de la source non biologique compte tenu de l'identification de la ou des substances,
ladite contamination étant en outre éventuellement une contamination biologique.

11. Appareil (1) d'identification d'une ou plusieurs substances dans un échantillon (3), comprenant :
une source d'éclairage configurée pour éclairer l'échantillon (3) avec une lumière d'une pluralité de modes d'excitation différents,
un détecteur (20) configuré pour mesurer l'intensité lumineuse provenant de l'échantillon (3) à une pluralité de longueurs d'onde pour obtenir un spectre Raman mesuré pour chacun des modes d'excitation, et
une unité de traitement (30) configurée pour identifier une ou plusieurs substances de l'échantillon (3) en utilisant conjointement les spectres mesurés ; étant entendu que :
les modes d'excitation sont de longueurs d'onde différentes,
et **caractérisé en ce que** :
les longueurs d'onde des modes d'excitation sont telles que les spectres mesurés comprennent au moins deux spectres parmi un spectre Raman non résonant, un spectre Raman pré-résonant et un spectre Raman résonant, et
l'unité de traitement (30) est configurée pour identifier la ou les substances au moyen des contributions aux spectres mesurés d'une pluralité de processus photophysiques de l'échantillon, dont la diffusion Raman de la lumière et la fluorescence.

12. Appareil (1) selon la revendication 11, dans lequel la source d'éclairage (10) comprend :
a) une source d'éclairage accordable en longueur d'onde, et/ou
b) une pluralité de sous-sources (12), chaque sous-source (12) étant configurée pour émettre de la lumière à une longueur d'onde différente des autres sous-sources (12).

13. Appareil (1) selon l'une quelconque des revendications 11 et 12, dans lequel :
a) la source d'éclairage (10) est configurée pour émettre une lumière polarisée, étant entendu éventuellement que
i) la polarisation de la lumière émise par la source d'éclairage (10) est accordable, et/ou
ii) la source d'éclairage (10) comprend une pluralité de sous-sources (12), chaque sous-source (12) étant configurée pour émettre une lumière ayant une polarisation différente des autres sous-sources (12), et/ou
b) la largeur de bande de la lumière émise par la source d'éclairage (10) est suffisamment étroite pour résoudre une largeur de raie Raman de 50 cm⁻¹ ou moins.

14. Appareil (1) selon l'une quelconque des revendications 11 à 13, dans lequel soit :
a) le détecteur (20) est configuré pour détecter une lumière réfléchie et/ou rétrodiffusée par l'échantillon (3), soit
b) le détecteur (20) est configuré pour détecter une lumière transmise à travers l'échantillon (3) et/ou diffusée par celui-ci.

15. Appareil (1) selon l'une quelconque des revendications 11 à 14, ledit appareil (1) comprenant en outre :
a) un élément de filtrage (50) configuré pour éliminer la lumière à la longueur d'onde du mode d'excitation de la lumière provenant de l'échantillon (3), ledit élément de filtrage (50) comprenant éventuellement un filtre optique passe-bas, un filtre optique passe-haut, un filtre optique coupe-bande dit « notch », un filtre optique passe-bande ou un modulateur électrooptique, et/ou
b) un polariseur de détection (52) configuré pour sélectionner une lumière ayant une polarisation prédéterminée parmi la lumière provenant de l'échantillon (3), et/ou
c) un élément de résolution spectrale (54) configuré pour résoudre spectralement la lumière provenant de l'échantillon (3), ledit élément de résolution spectrale (54) comprenant éventuellement un spectrographe, une grille, un prisme ou un interféromètre.
